# EUROPEAN PATENT SPECIFICATION

(11) **EP 2 506 835 B1**
(45) Date of publication and mention of the grant of the patent: **12.06.2019**
(21) Application number: 10833860.9
(22) Date of filing: 23.11.2010
(51) Int. Cl.: A61K 9/16, A61K 9/20, A61K 9/50

(54) **COMPRESSIBLE-COATED PHARMACEUTICAL COMPOSITIONS AND TABLETS AND METHODS OF MANUFACTURE**
KOMPRIMIERBARE BESCHICHTETE PHARMAZEUTISCHE ZUSAMMENSETZUNG SOWIE TABLETTEN DARAUS UND HERSTELLUNGSVERFAHREN DAFÜR
COMPOSITIONS PHARMACEUTIQUES ENROBÉES ET COMPRESSIBLES, COMPRIMÉS ET PROCÉDÉS DE FABRICATION ASSOCIÉS

(30) Priority: 30.11.2009 US 265213 P
(43) Date of publication of application: 10.10.2012
(73) Proprietor: Adare Pharmaceuticals, Inc., Lawrenceville, NJ 08648 (US)
(72) Inventor: VENKATESH, Gopi, M., Vandalia, Ohio 45377 (US); LAI, Jin-Wang, Springboro, Ohio 45066 (US); CLEVENGER, James, M., Vandalia, Ohio 45377 (US); KRAMER, Craig, New Lebanon, Ohio 45345 (US)
(74) Representative: Cooley (UK) LLP
(86) International application number: PCT/US2010/057811
(87) International publication number: WO 2011/066287

(56) References cited:
- WO-A1-2010/127346
- US-A- 5 004 595
- US-A1- 2006 039 975
- US-A1- 2009 169 620
- US-A1- 2009 202 630
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368

## Description

### BACKGROUND OF THE INVENTION

Although two of the most widely used dosage forms are tablets and capsules, such dosage forms have several disadvantages. For example, an estimated 50% of the population has problems swallowing tablets (see Seager in Journal of Pharmacol. and Pharm. 50, pages 375-382, 1998); it is especially hard for aged persons to swallow tablets or capsules or to medicate children who are unable or unwilling to swallow tablets or capsules. This leads to poor compliance, even non-compliance, with the treatment and thus has a negative impact on the efficacy of the treatment. Many drugs are bitter, which precludes the medication from being easily sprinkled onto food such as applesauce, a commonly used method of administering medications to children. The conventional capsule or tablet dosage form is also inconvenient for the "people on the move" who often do not have access to drinking water or fluids.

Orally disintegrating dosage forms have steadily grown in popularity as more convenient and potentially safer alternatives to conventional tablets and capsules. These rapidly disintegrating dosage forms disintegrate or dissolve in the oral cavity, and they are easily swallowed without water. They are a boon to individuals who have difficulty swallowing conventional tablets (common among geriatric and pediatric patients); people who do not have ready access to water (e.g., bed-ridden or immobile patients, or active people often away from home); and caregivers whose patients are reluctant to take their medications. Orally disintegrating dosage forms help to improve patient compliance with oral dosage regimens because they are easy to administer, convenient to take discreetly anywhere, and difficult to discard once administered.

The drug particles need to be small enough, coated with one or more polymers for effective taste masking, and formulated into an orally disintegrating tablet ("ODT") such that the ODT rapidly disintegrates in the oral cavity of the patient creating a smooth easy-to swallow suspension containing coated drug particles with a non-gritty mouthfeel and aftertaste. Drug-containing particles suitable for incorporation into orally disintegrating tablets must exhibit one or more of the following characteristics:
- Coated with one or more polymers at an appropriate coating level to achieve desirable organoleptic properties (e.g., none or insignificant drug release in the oral cavity for effective taste-masking and aftertaste).
- A desired average particle size (e.g., typically< 500 µm) to achieve a non-gritty mouthfeel.
- Desired pharmacokinetic properties (i.e., *in vitro* drug release, plasma concentration-time profile, Cₘₐₓ, Tₘₐₓ, terminal elimination half life, and AUC).

Microcapsules are small particles (typically < 400 µm in average diameter) encapsulated by coating layers comprising one or more polymers or fatty acids and/or esters that are thick enough to prevent drug release in the oral cavity, i.e., in actuality, the coating layers effectively mask the taste of the underlying drug and are particularly suitable for formulations of ODTs. In such cases, it is necessary to insure that the coated drug particles provide desired drug release profiles under *in vitro* and *in vivo* conditions. In case of immediate-release (IR) dosage forms, the desired *in vitro* drug-release / pharmacokinetic properties (i.e., rapid drug release, Cₘₐₓ, and AUC) need to be similar to the RLD (reference listed drug) to be bioequivalent. In contrast, the ODTs comprising controlled-release drug particles must exhibit desired *in vitro* drug-release / pharmacokinetic properties [e.g., sustained-release (SR), bimodal {IR+ SR, IR+ TPR (timed pulsatile release), or IR+ TSR (timed sustained release)} profiles, plasma concentration-time profiles, Cₘₐₓ, Tₘₐₓ, plasma elimination half-life, and AUC] to be suitable for a once-or twice-daily dosing regimen.

It can be challenging to coat small drug-containing particles to achieve a balance between effective taste-masking and rapid drug release to be bioequivalent, in the case of IR dosage forms, or prolonged *in vitro* drug-release profiles, in the case of CR dosage forms using Microcaps® (microencapsulation) or Diffucaps® (fluid-bed coating) technology. This is particular true when the drug is extremely bitter, freely soluble in water or gastrointestinal fluids and/or has a pH dependent solubility profile. One approach in accordance with the disclosures of US 6,139,865 or the co-pending Patent Application Ser. No. 10/827,106 filed Apr. 19, 2004 (Publication No. U.S. 2005/0232988), No. 11/213,266 filed Aug. 26, 2005 (Publication No. U.S. 2006/0105038); No. 11/256,653 filed Oct. 21, 2005 (Publication No. U.S. 2006/0105039); No. 11/248,596 filed Oct. 12, 2005 (Publication No. U.S. 2006/0078614), and No. 12/370,852 filed Feb 13, 2009 (Publication No. U.S. 2009/0202630), has been to coat drug-containing particles (drug crystals, drug granules, drug pellets, or drug layered beads) with thick layer(s) comprising from about 10% to 65% w/w of water-insoluble polymer (e.g., ethylcellulose with a mean viscosity of 100 cps) alone or in combination with a gastrosoluble organic, inorganic or polymeric pore-former by solvent coacervation or fluid-bed coating to insure that the bitter API will not be exposed while in the oral cavity. Another approach based on the disclosures in US 6,627,223; US 7,387,793, or the co-pending U.S. Patent Application Ser. Nos. 11/668,408 filed Jan. 29, 2007 (Publication No. U.S. 2008/0196491), 11/847,219 filed Aug. 29, 2006 (Publication No. U.S. 2007 /0069878), and 12/424,201 filed April 15, 2009 (Publication No. U.S. 2009/0258066), and PCT Publications 2010/096820 and WO 2010/096814 is to produce SR or TPR beads exhibiting sustained-release or timed, pulsatile-release profiles. Furthermore, it is a requirement that coated drug-containing particles (e.g., taste-masked, SR-coated or TPR coated microparticles) with a mean particle size of not more than 500 µm for incorporation into an ODT, such that said ODT rapidly disintegrates in the oral cavity into a smooth, easy to-swallow suspension, exhibit a non-gritty mouthfeel and no aftertaste. As a consequence, bitter drugs requiring rapid release in the GI tract present unique challenges in formulating orally dissolving dosage forms. Bowen (2002; "Particle size distribution measurement from millimeters to nanometers and from rods to platelets" Journal of Dispersion Science and Technology vol.23 (5) 631-662) describes various methods of obtaining particles of a desired size, and methods of measuring particle size.

Lastly, orally disintegrating tablets are typically compressed at low compression forces to achieve rapid disintegration in the oral cavity or when tested in accordance with USP disintegration time test. Consequently, OTDs are more friable than the conventional tablets. Tablet hardness and friability can be improved by including a compression aid such as microcrystalline cellulose in the tablet matrix. However, this results in a chalky mouthfeel. Furthermore, robust tablet formulations exhibiting acceptable tablet hardness and friability are also required for bulk packaging and/or packaging in HDPE bottles or push through blisters (most preferred packaging), for transportation, commercial distribution, and end use. Although ODTs were introduced into the market in the 1980s, these challenges have not been adequately addressed.

WO2010/1276346 describes pharmaceutical compositions comprising a plurality of taste-masked non-opioid analgesic/opioid analgesic drug-containing microparticles, dosage forms comprising such pharmaceutical compositions (such as an orally disintegrating tablet), and methods of making the pharmaceutical compositions and dosage forms.

US 5,004,595 describes a free-flowing particulate delivery system for providing enhanced flavor and sweetness to comestible compositions comprising a powdered flavor composition encapsulated in a matrix comprising an outer hydrophilic coating containing up to the solubility limit of the coating of an intense sweetener. The delivery system is particularly useful in chewing gums, confectioneries and pharmaceutical preparations as well as other food products.

US2009/169620 discloses orally disintegrating tablet compositions comprising a therapeutically effective amount of at least one drug such as temazepam, 0.5-3% of an ODT binder polymer, a sugar alcohol and/or saccharide, and a disintegrant.

From a pharmaceutical and practical point of view, the inventors of the present invention have examined various methods of improving tableting properties (e.g., higher hardness and lower friability) of OTDs comprising heavily coated drug-containing microparticles, without sacrificing organoleptic properties (e.g., effective taste-masking, non-gritty mouthfeel and no aftertaste). The method of applying a compressible coating layer comprising a non-polymeric water-soluble compressible sweetener such as Sucralose, directly disposed over polymer-coated drug-containing microparticles for effectively taste-masking and rapid release, sustained-release or timed, pulsatile-release properties prior to their incorporation into OTDs, was surprisingly found to result in OTDs with significantly improved tableting properties even though compressed at significantly lower compression forces.

### SUMMARY OF THE INVENTION

The invention relates to multiparticulate pharmaceutical compositions comprising coated microparticles comprising one or more drugs wherein the coated particles are further coated with a compressible coating agent for improving tableting properties and methods for preparing pharmaceutical compositions comprising compressible coated microparticles and orally disintegrating tablets.
The invention provides a pharmaceutical composition comprising compressible coated microparticles comprising:
a. drug particles comprising a drug and/or a pharmaceutically acceptable salt, ester, polymorph, or solvate thereof;
b. at least one modified-release membrane layer comprising a water-insoluble polymeric material disposed over the drug-containing cores; and
c. a compressible coating layer comprising a non-polymeric sweetener, disposed directly over the modified release coated drug particles from (b).

In another embodiment, the present invention is directed to a pharmaceutical composition comprising a therapeutically effective amount of drug-containing particles, comprising one or more membrane layers to effectively mask the taste as well as the aftertaste of the drug and to provide desired pharmacokinetics profile upon oral administration to a patient in need of medication. In another embodiment, the present invention is directed to a pharmaceutical composition comprising a therapeutically effective amount of drug-containing particles, comprising one or more membrane layers to effectively mask the taste as well as to provide rapid release of the dose upon entry into the stomach to be bioequivalent to the reference listed immediate release (IR) drug product. In certain other embodiments, the present invention is directed to a pharmaceutical composition comprising a drug comprising one or more membrane layers to effectively mask drug taste as well as to provide a controlled-release profile (e.g., a sustained-release (SR), timed, pulsatile release (TPR), timed, sustained release (TSR) or modified release (IR+ TPR,-IR + SR, SR+ TPR or IR+ TSR)) to be suitable for a once- or twice-daily dosing regimen, i.e., in other words, the present invention is directed to a pharmaceutical composition comprising a drug comprising one or more membrane layers not only to effectively mask the drug taste but also to provide a controlled-release (CR) profile, thereby improving patient compliance. In each of the cases, the coated drug-containing microparticles are further coated with a non-polymeric compressible sweetener such as sucralose, lactitol, sorbitol, or maltitol to minimize or eliminate membrane fracture during tableting of the compression blend comprising taste masked and/or controlled release coated microparticles, rapidly dispersing microgranules, and other ODT excipients including one or more flavors, a sweetener, etc., wherein the ODT tablet thus produced rapidly disintegrates in the oral cavity forming a smooth, easy-to-swallow suspension exhibiting non-gritty mouthfeel and no aftertaste.

The invention also provides an orally disintegrating tablet (ODT) or a rapidly dispersing tablet (RDT) composition comprising a taste-masked and/or modified-release coated drug-containing cores of the invention comprising:
a. drug particles comprising a therapeutically effective amount of a drug and/or a pharmaceutically acceptable salt, ester, or solvate thereof;
b. one or more polymeric membranes, each membrane comprising a water-insoluble polymer or optionally a water-insoluble and gastrosoluble polymeric blend material;
c. a compressible coating with a non-polymeric sweetener selected from the group consisting of sucralose, lactitol, sorbitol, maltitol, or a mixture thereof, directly disposed over modified release coated drug particles of step (b.); and
d. rapidly dispersing granules comprising (i) a disintegrant and (ii) a sugar alcohol, a saccharide, or a mixture thereof, at a ratio of from 90:10 to 99:1.

In certain other embodiments, the present invention is directed to a taste-masked composition comprising a first coating layer comprising a coacervated polymeric material, and optionally a second coating layer comprising a fluid-bed coated blend polymeric material comprising a combination of a water-insoluble polymer and a water-soluble or gastrosoluble pore-forming polymer to produce taste-masked microparticles. The taste-masked and/or controlled release microparticles are further coated with a compressible coating layer of lactitol, and the compressible coated drug particles are blended with other pharmaceutically acceptable excipients (e.g., a diluents, compression aide, lubricant, etc.) and compressed into rapidly dispersing tablets that rapidly disperse into coated microparticles on contact with water or body fluids which control drug release and pharmacokinetic profiles.

The invention also provides a method of manufacturing a taste-masked composition comprising the steps of:
a.
   (i) applying one or more polymeric membranes onto drug particles containing a drug and/or a pharmaceutically acceptable salt, solvate, polymorph, or ester thereof, with a desired particle size distribution; or
   (ii) applying a first coating layer comprising a water-insoluble polymeric material for a weight gain of from 5% w/w to 20% w/w, onto microparticles containing a drug and/or a pharmaceutically acceptable salt, solvate, polymorph, or ester thereof, with desired particle size specifications; and
      applying an optional second coating layer comprising a water insoluble-enteric polymer blend material at a ratio of water insoluble polymer to enteric polymer of from 9:1 to 1:2 for a weight gain of from 10% w/w to 40% w/w,
      onto drug particles
b. a compressible coating with a non-polymeric material directly disposed over the modified-release coated drug particles of step (a);
c. producing rapidly dispersing granules with an average particle size of not more than 400 µm, comprising a sugar alcohol, a saccharide, or a mixture thereof, and a super disintegrant with an average particle size of not more than 30 µm, by granulating said mixture at a ratio of from 99/1 to 90/10 using water as a granulating fluid and drying in a fluid bed dryer or in a tray drying;
d. blending compressible coated taste-masked drug particles from (b), rapidly dispersing micro granules from (c) and additional excipients comprising one or more flavoring agents, one or more colorants, a sweetener, additional disintegrant, and a diluent such as microcrystalline cellulose; and
e. compressing the blend from (d) into orally disintegrating tablets using a rotary tablet press equipped with an external lubrication system,
wherein
(1) one or more said taste-masking layers effectively mask the taste of said drug and/or the pharmaceutically acceptable salt, ester, or solvate thereof,
(2) said orally disintegrating tablet rapidly disintegrates in the oral cavity of a patient into a smooth, easy-to-swallow suspension containing taste-masked particles exhibiting non-gritty mouthfeel and no aftertaste.

These and other embodiments, advantages and features of the present invention become clear from the detailed description and examples provided in subsequent sections.

### BRIEF DESCRIPTION OF THE DRAWINGS

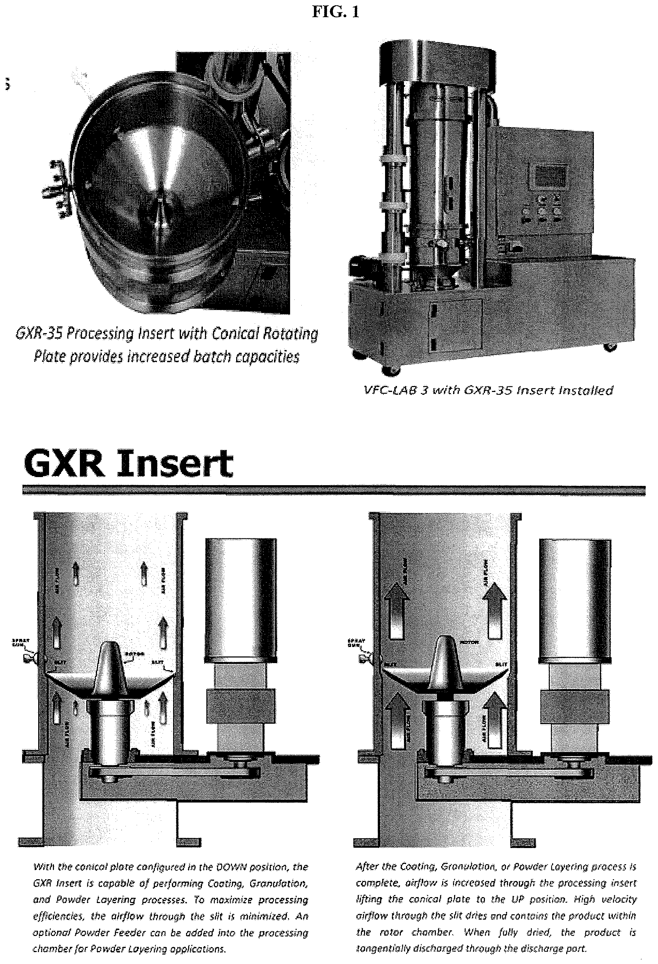

- **FIG. 1**: shows Granurex GRX-35 Insert mounted on VFC-Lab 3
- **FIG. 2**: shows the histograms: QICPIC Particle Size Analysis of lbuprofen Pellets of

### Example 3.

### DETAILED DESCRIPTION OF THE INVENTION

The term "drug," "active," "therapeutic agent" or "active pharmaceutical ingredient" as used herein includes a pharmaceutically acceptable and therapeutically effective amount of the drug such as ranitidine or the pharmaceutically acceptable salts, stereoisomers and mixtures of stereoisomers, solvates (including hydrates), polymorphs, and/or esters thereof (e.g., of ranitidine). When referring to a drug such as ranitidine in the descriptions of the various embodiments of the invention, the reference also encompasses pharmaceutically acceptable salts, stereoisomers and mixtures of stereoisomers, solvates (including hydrates), polymorphs, and/or esters of said drug.

The terms "orally disintegrating tablet," "orally dissolving tablet," "orally dispersing tablet" or "ODT" refer to a solid dosage form of the present invention, which disintegrates rapidly in the oral cavity of a patient after administration, without chewing. The rate of disintegration can vary but is faster than the rate of disintegration of conventional solid dosage forms or chewable solid dosage forms (e.g., tablets) which are intended to be swallowed immediately with water or chewed after administration. Orally disintegrating compositions of the present invention can contain pharmaceutically acceptable ingredients which swell, dissolve or otherwise facilitate the disintegration or dissolution of the ODT composition. Such ingredients can include pharmaceutical disintegrant such as crospovidone, water-soluble sugar alcohol such as mannitol, a saccharide such as lactose, or a mixture thereof, a water-soluble binder such as povidone, a meltable solid (e.g., a wax) polyethylene glycol, which can release the drug upon entering the stomach. Orally disintegrating compositions of the present invention may be in the form of a tablet or minitablet.

The term "about," as used herein to refer to a numerical quantity, includes "exactly". For example, "about 60 second" includes 60 seconds, exactly, as well as values close to 60 seconds (e.g., 50 seconds, 55 seconds, 59 seconds, 61 seconds, 65 seconds, 70 seconds, etc.).

The term "core" includes but is not limited to a bead, pellet, microgranule, granulate, mini-tablet, drug crystal, etc., having a size typically in the range of from about 100 µm to about 800 µm, about 100 µm to about 600 µm, about 100 µm to about 500 µm, about 100 µm to about 400 µm, about 200 µm to about 600 µm, about 200 µm to about 500 µm, about 200 µm to about 400 µm, about 300 µm to about 500 µm, about 300 µm to about 600 µm and subranges therebetween.

As used herein, the term "modified-release" coating encompasses coatings that include taste-masking that delay release, sustain release, extend release, prevent release, and/or otherwise prolong the release of a drug relative to formulations lacking such coatings which release a drug relatively quickly (i.e., "immediate release" compositions). The term "controlled-release" encompasses "sustained release," "extended release," "delayed release," and "timed, pulsatile release." The term "lag-time" coating refers to a particular type of "controlled release" coating in which the lag time coating delays release of a drug after administration. The term "controlled release" is also used interchangeably with "modified release." The term "controlled-release particle" refers to a particle showing one or more controlled-release properties, as described herein. The term "controlled-release particle" also refers to a drug-containing particle coated with one or more controlled-release coatings, as described herein.

The term "lag time" refers to a time period immediately after administration of the drug-containing particle wherein less than about 10%, for example less than about 9%, less than about 8%, less than about 7%, less than about 6%, less than about 5%, less than about 4%, less than about 3%, less than about 2%, less than about 1 %, or more substantially about 0%, of the drug is released from a particle. In the context of *in vitro* dissolution testing, lag time refers to the time period immediately after exposure to dissolution conditions, wherein less than about 10%, for example less than about 9%, less than about 8%, less than about 7%, less than about 6%, less than about 5%, less than about 4%, less than about 3%, less than about 2%, less than about 1 %, or more substantially about 0%, of the drug is released from the drug-containing particle.

As used herein, the term "immediate release" (IR) refers to release of greater than or equal to about 50% (especially if taste-masked for incorporation into an orally disintegrating tablet), in some embodiments greater than about 75%, in other embodiments greater than about 90%, and in still other embodiments greater than about 95% of the drug within about 2 hours, or in other embodiments within about one hour following administration of the dosage form.

As used herein, the term "immediate-release core" refers to a core as defined herein comprising a drug and an alkaline agent, optionally layered with a sealant layer, wherein the optional sealant layer functions to protect the immediate-release core from attrition and abrasion, but does not provide any substantial controlled-release properties. An "immediate-release core" can include drug crystals (or amorphous particles); an alkaline agent and granules or granulates of the drug with one or more excipients, an inert core (e.g., a sugar sphere) layered with a drug (and an optional binder), an optional protective sealant coating, and an alkaline buffer layer, or an alkaline agent layered with a drug (and an optional binder), and an optional protective sealant coating. Immediate-release cores have immediate release properties as described herein. Controlled-release particles (e.g., extended-release particles; sustained-release particles; delayed-release particles; timed, pulsatile release-particles, etc.) can be prepared by coating immediate-release cores with one or more controlled-release coatings.

As used herein, the term "sustained-release" (SR) refers to the property of slow release of a drug from a drug-containing core particle, without an appreciable lag time. The term "sustained-release coating" or "SR coating" refers to a coating showing sustained-release properties. The term "sustained-release particle" refers to a drug-containing particle showing sustained-release properties. In one embodiment, a sustained-release coating comprises a water-insoluble polymer and optionally a water-soluble polymer. An SR coating can optionally contain a plasticizer or other ingredients that do not interfere with the "sustained-release" properties of the coating.

As used herein, the term "timed, pulsatile release" (TPR) refers to the property of modified release of a drug after a pre-determined lag time. The term "timed, pulsatile-release coating" or "TPR coating" refers to a coating showing timed, pulsatile-release properties. The term "timed, pulsatile-release particle" refers to a drug-containing particle showing timed, pulsatile-release properties. In some embodiments, a lag time of from at least about 2 to about 10 hours is achieved by coating the particle with, e.g. a combination of at least one water-insoluble polymer and at least one enteric polymer (e.g., a combination of ethylcellulose and hypromellose phthalate). A TPR coating can optionally contain a plasticizer or other ingredients which do not interfere with the "timed, pulsatile release" properties of the coating.

As used herein, the term "delayed release" (DR) refers to the property of immediate release of a drug after a predetermined lag time. The term "delayed release coating" or "DR coating" refers to a coating showing delayed-release properties. The term "delayed release particle" refers to a drug-containing particle showing delayed-release properties. In some embodiments, a drug-containing particle showing delayed-release properties such that no substantial drug release occurs until exposure to an alkaline pH, is achieved by coating the particle with an enteric polymer (e.g., hypromellose phthalate). A delayed-release coating can optionally contain a plasticizer or other ingredients which do not interfere with the delayed-release properties of the coating.

The term "disposed over", e.g. in reference to a coating over a substrate, refers to the relative location of e.g. the coating in reference to the substrate, but does not require (unless clearly stated to the contrary) that the coating be in direct contact with the substrate. For example, a first coating "disposed over" a substrate can be in direct contact with the substrate, or one or more intervening materials or coatings can be interposed between the first coating and the substrate. In other words, for example, an SR coating disposed over a drug-containing core can refer to an SR coating deposited directly over the drug-containing core, or can refer to an SR coating deposited onto a protective seal coating deposited on the drug-containing core.

The terms "sealant layer" or "seal coating" refer to a protective membrane disposed over a drug-containing core particle such as a drug-layered bead.

The term "substantially disintegrates" refers to a level of disintegration amounting to disintegration of at least about 50%, at least about 60%, at least about 70%, at least about 80%, at least about 90%, or about 100% disintegration. The term "disintegration" is distinguished from the term "dissolution," in that "disintegration" refers to the breaking up of or loss of structural cohesion of e.g. the constituent particles comprising a tablet, whereas "dissolution" refers to the solublization of a solid in a liquid (e.g., the solublization of a drug in solvents or gastric fluids).

The term "substantially masks the taste" in reference to the taste-masking layer of IR particles in a dosage form (when present), refers to the property of the taste-masking layer of substantially preventing the release or dissolution of the drug in the oral cavity of a patient, thereby preventing the patient from tasting the drug. A taste-masking layer which "substantially masks" the taste of the drug typically releases less than about 10% of the drug in the oral cavity of the patient, in other embodiments, less than about 5%, less than about 1 %, less than about 0.5%, less than about 0.1 %, less than about 0.05%, less than about 0.03%, or less than about 0.01 % of the drug. The taste-masking properties of the taste-masking layer of the compositions of the present invention can be measured *in vivo* (e.g., using conventional organoleptic testing methods known in the art) or *in vitro* (e.g., using dissolution tests as described herein). The skilled artisan will recognize that the amount of drug release associated with a taste-masking layer that "substantially masks" the taste of a drug is not limited to the ranges expressly disclosed herein, and can vary depending on other factors such as the perceived bitterness of the drug and, e.g. the presence of flavoring agents in the composition.

The term "substantially free" means that the ingredient indicated is not present, or is present in only insignificant amounts. In one embodiment, "substantially free" means less than about 10%. In other embodiments, "substantially free" means less than about 5%, less than about 2%, or less than about 1 %, or about 0%. For example, a coating that is substantially free of water-insoluble polymers does not contain any water-insoluble polymer in a substantial amount. The term "substantially free of water-insoluble polymers" does not exclude polymers that are water-soluble or water-insoluble ingredients that are not polymers.

The term "water insoluble" means insoluble or very sparingly soluble in aqueous media, independent of pH, or over a broad physiologically relevant pH range (e.g., pH 1 to about pH 8). A polymer that swells but does not dissolve in aqueous media can be "water insoluble," as used herein.

The term "water soluble" means soluble (i.e., a significant amount dissolves) in aqueous media, independent of pH. An ingredient that is soluble over a limited pH range could be (but is not necessarily) considered "water soluble," as used herein. An ingredient that is "water soluble" (as used herein) does not contain ionizable functional groups; i.e., functional groups that ionize over a change in pH. For example, a polymer that is soluble under ^{∼} neutral to alkaline conditions, or soluble at pH 5 .5 and above, pH 6 and above, or about pH 7.0, and is insoluble under lower pH conditions could be (but is not necessarily) considered "water soluble," as used herein.

The term "enteric" or "enterosoluble" means soluble (i.e., a significant amount dissolves) under intestinal conditions; i.e., in aqueous media under alkaline conditions and insoluble under acidic conditions (i.e., low pH). For example, an enteric polymer that is soluble under neutral to alkaline conditions and insoluble under low pH conditions is not necessarily "water soluble," as used herein. Polyvinyl acetate phthalate, soluble at pH 4.5 and above, is an example of an enteric polymer.

The term "reverse enteric" means soluble under acidic conditions and insoluble under neutral to alkaline conditions. A reverse enteric polymer is not considered "water soluble" as used herein.

The term "gastrosoluble pore-former" refers to a pore-former which is insoluble at neutral to alkaline pHs, but is readily soluble under acidic conditions. An organic pore-former (e.g., calcium saccharide, calcium succinate), inorganic pore-former (e.g., calcium carbonate, magnesium oxide), or polymeric pore-former (e.g., Eudragit EPO or AEA ®) can be utilized as a gastrosoluble pore-former in accordance with the present invention.

The term "rapidly dispersing tablet" refers to a tablet in which taste-masked or controlled release microparticles (e.g. drug containing cores such drug crystalline particles, drug layered onto inert cores, and spheronized or powder layered pellets coated with at least one taste-masking polymeric membrane layer or at least one water-insoluble, sustained release polymer layer) are embedded in a excipient matrix, that rapidly disperses on contact with water and/or body fluid. The membrane disposed on said dispersed microparticles controls the drug release. Although these tablets are meant to be swallowed, there are at least two other modes of oral administration for subjects or patients in need of medication who experience difficulty swallowing a tablet or a capsules - breaking a rapidly dispersing tablet into two halves for ingestion of one half, then the other, and rapidly dispersing the tablet in about 150 mL of water, swirling and drinking the drug preparation.

The terms "plasma concentration vs. time profile," "Cmax," "AUC," "T max," and "elimination half life" have their generally accepted meanings as defined in the FDA Guidance for Industry: Bioavailability and Bioequivalence Studies for Orally Administered Drug Products - General Considerations (issued March 2003).

In one embodiment, the orally disintegrating compositions of the present invention comprise a therapeutically effective amount of a highly spherical drug particles, such as ranitidine hydrochloride, coated with at least one taste-masking layer and a compressible coating layer directly disposed over said taste-masked microparticle, and in the form of an orally disintegrating tablet (ODT) further comprising rapidly dispersing microgranules comprising a disintegrant and a sugar alcohol, a saccharide or a mixture thereof. Upon administration of an orally disintegrating composition in an oral dosage form of the present invention (e.g., an ODT) to the oral cavity of a patient, the oral dosage form (e.g., the tablet) disintegrates rapidly in the patient's oral cavity while the rapidly dispersing microgranules dissolve into a smooth easy-to-swallow suspension containing taste-masked drug particles.

The rate of disintegration of orally disintegrating compositions in the oral cavity of a patient can be on the order of about 60 seconds or less, about 50 seconds or less, about 40 seconds or less, about 30 seconds or less, about 20 seconds or less, or about 10 seconds or less.

Alternatively, the rate of disintegration of the orally disintegrating compositions of the present invention can be measured using various *in vitro* test methods, for example the USP <701> Disintegration Test. When using the USP <701> Disintegration Test, the rate of disintegration of orally disintegrating compositions is faster than that of conventional oral, non-orally disintegrating compositions, for example about 60 seconds or less, about 30 seconds or less, about 20 seconds or less, or about 10 seconds or less.

The term "drug dissolution profile" refers to the dissolution profile of a drug-containing composition. The rate of dissolution of orally disintegrating compositions of the present invention comprising taste-masked IR microparticles can be evaluated using the United States Pharmacopoeia Apparatus 2 (paddles@ 50 or 75 rpm in 900 mL of 0.1N HCl or pH=4.5, 5.8 or 6.8 buffer). When using the United States Pharmacopoeia Apparatus 2 test, the rate of dissolution of the drug (e.g., ranitidine) needs to be comparable to that of the conventional, non- orally disintegrating composition (i.e., the reference listed drug (RLD) immediate-release product (e.g., Zantac®)), for example about 70% or more, about 75% or more, about 80% or more, about 85% or more, about 90% or more, about 95% of the total amount of drug is released in 45 min.

The term "non-orally disintegrating immediate-release drug composition" refers to non-orally disintegrating compositions containing said drug such as conventional tablets or capsules intended to be swallowed and absorbed in the gastrointestinal tract, chewable tablets which require mastication in order to break apart the tablet structure, known in the art.

Thus, an orally disintegrating composition comprising immediate-release (IR) or taste-masked drug particles of the present invention will have plasma concentration-time profiles substantially similar to that of the non-orally disintegrating immediate-release composition, and pharmacokinetics (PK) parameters, AUCco-24) and Cmax, will be within the 90% confidence interval (CI) of 80.0% -125.0% of the respective values for the RLD product, such as Zantac, dosed under identical conditions in a properly conducted crossover PK study, to be bioequivalent to the marketed product.

Alternatively, the rate of drug release from multi-coated drug-containing particles constituting the orally disintegrating compositions of the present invention can be evaluated using the United States Pharmacopoeia Apparatus 2 (paddles @ 50 rpm at 37°C and a 2-stage dissolution media (e.g., in 700 mL of 0.IN HCl buffer for the first 2 hours and testing thereafter in 900 mL of pH=6.8 buffer).

A microparticle as used in the present invention refers to a particle or a granule with an average particle size of not more than about 500 µm, more particularly not more than about 400 µm. The terms "particle," "microparticle," "granule" and/or "microgranule" are herein used interchangeably to mean a particle with a mean particle size of not more than about 500 µm, irrespective of whether said particle contains a drug and/or a sugar alcohol or not. The term "microcaps" refers to specifically taste-masked drug-containing particles with a mean particle size of not more than about 500 µm.

The microparticles can be described as primary particles or secondary particles. Primary particles are unagglomerated, whereas secondary particles are agglomerated primary particles. Thus, primary particles rapidly dispersing micro granules comprising a sugar alcohol, a saccharide, or a mixture thereof (e.g., D-mannitol with an average particle size or diameter of not more than 30 µm) and a disintegrant (e.g., Crospovidone XL-IO with an average particle size or diameter of not more than 30 µm) are generally smaller than secondary particles (e.g., rapidly dispersing micro granules with an average particle size or diameter of not more than 400 µm).

Unless indicated otherwise, all percentages and ratios are calculated by weight. Unless indicated otherwise, all percentages and ratios are calculated based on the total composition.

The orally disintegrating compositions of the present invention may have one or more of the following advantages: palatable drug formulations with good disintegration characteristics and pharmacokinetics; improved patient compliance for patients who have difficulty swallowing conventional tablets; and easy and/or convenient administration by the patient or the patient's caregiver.

Ideally an orally disintegrating composition should be palatable, e.g. have acceptable taste and mouthfeel characteristics. For bitter tasting drugs such as ranitidine, the orally disintegrating formulation may include a taste-masking polymer to improve the taste characteristics of the formulation, as well as a disintegrant, a sugar alcohol, a saccharide, or a mixture thereof, to provide rapid disintegration in the oral cavity as well as a "creamy" mouthfeel. In addition, the orally disintegrating formulation must also provide acceptable pharmacokinetics and bioavailability to provide the desired therapeutic effect. These desired properties of an orally disintegrating formulation can be contradictory, in that components of the formulation that provide acceptable taste-masking properties can inhibit or delay release of the drug, thereby providing unacceptable pharmacokinetic properties. Conversely, components of the formulation that promote release of the drug in the oral cavity result in undesirable taste or mouthfeel properties. Accordingly, an acceptable orally disintegrating tablet formulation designed to be bioequivalent to the IR drug product should balance these contradictory characteristics in order to provide a palatable (e.g., well taste-masked), fast disintegrating tablet composition with acceptable pharmacokinetics (e.g., rapid drug dissolution upon entry into the stomach). Further more, these tablets are required to possess sufficient hardness and low friability to withstand rigors of attrition when packaged in bulk containers, HDPE bottles or in push-through blisters for transportation, commercial distribution, and end use. However, the drug-containing particles heavily coated with one or more water-insoluble polymers for effective taste-masking and/or prolonged drug release are typically rigid and consequently form friable tablets, especially when compressed into OTDs at low compression forces. Hence, the polymer-coated drug particles require a compressible coating with a compressible coating material (e.g., sucralose) disposed over drug-containing particles to achieve the most desirable tableting properties including structural integrity, i.e., avoiding cracks or membrane fracture during tablet compression.

The compositions of the present invention can comprise any combination of therapeutically effective amounts of one or more drugs, taste-masking polymers, and one or more pharmaceutically acceptable ingredients which provide an orally disintegrating composition as defined herein. For example, ranitidine hydrochloride drug substance with a desired particle size range [e.g., not more than 5% retained on 30 mesh (600 µm) screen and not more than 10% through 270 mesh screen (53 µm)] are microencapsulated with a water-insoluble polymer by solvent coacervation in accordance with the disclosures of US 6,139,863 and co-pending U.S. Patent Application Ser. No. 10/827,106 filed Apr. 19, 2004 (Publication No. U.S. 2005/0232988). These taste-masked particles further coated with water-soluble sucralose are combined with granules comprising a disintegrant, a sugar alcohol and/or a saccharide granulated with purified water in a high shear granulator and dried in a tray drying conventional oven or in a fluid bed dryer (this material is hereafter referred to as rapidly dispersing micro granules), and compressed into orally disintegrating tablets sufficiently strong to withstand the rigors of transportation in bulk containers or HDPE bottles, whereby the disintegrant, sugar alcohol or saccharide swells and/or dissolves in the saliva of a patient's oral cavity, thereby forming a smooth, easy-to-swallow suspension containing taste-masked or CR-coated drug particles. Additionally, other ODT excipients such as one or more flavoring agent such as a cherry or a mint flavor, a sweetener such as sucralose, additional disintegrant (the same or a different disintegrant) to promote rapid disintegration, and optionally one or more colorants, are included and to further improve organoleptic properties of the orally disintegrating tablet formulation.

In certain embodiments, the present invention relates to a pharmaceutical composition comprising one or more populations of controlled-release microparticles, wherein each microparticle comprises a core comprising at least one drug or a pharmaceutically acceptable salt, solvate, and/or ester thereof, a first coating disposed over said core, comprising a water-insoluble film-forming material (e.g., ethylcellulose or a fatty acid ester) and an outer coating directly disposed over said core, comprising a compressible coating material (e.g., water-soluble sucralose) wherein said compressible coating material comprising a non-polymeric sweetner, to achieve significantly improved tableting properties.

In one embodiment, the present invention relates to a pharmaceutical composition comprising a first or second population of controlled-release particles, wherein each controlled-release particle comprises a core comprising at least one drug or a pharmaceutically acceptable salt, solvate, and/or ester thereof; a water-insoluble polymer (e.g., ethylcellulose), a second optional coating disposed over the first coating, comprising a water-insoluble polymer in combination with an enteric polymer (e.g., ethylcellulose and hypromellose phthalate at a ratio of from about 9: 1 to about 1 :2), and a third coating directly disposed over the second coating comprising a compressible coating material (e.g., water-soluble sucralose) wherein the coating material is substantially free of a polymer.

In certain other embodiments as disclosed in U.S. Patent Application Ser. Nos. 11/668,408 filed Jan. 29, 2007, (Publication No. U.S. 2008/0196491), 11/847,219 filed Aug. 29, 2007, (Publication No. U.S. 2008/0069878), and 12/424,201, filed April 15, 2009, (Publication No. U.S. 2009/0258066), as well as PCT Publications WO 2010/096820 and WO 2010/096814the invention relates to a pharmaceutical composition comprising one or more populations of controlled-release microparticles, wherein each microparticle comprises a core comprising at least one drug or a pharmaceutically acceptable salt, solvate, ester, and/or a mixture thereof, a first coating disposed over said core, comprising a water-insoluble film-forming material (e.g., ethylcellulose or a fatty acid ester) and/or an optional second coating comprising a water-insoluble polymer in combination with an enteric polymer, and an outer coating layer directly disposed over said CR-coated core, wherein said compressible coating material is a hydrophilic polymer such as hydroxypropylcellulose. The orally disintegrating tablets comprising compressible polymer coated drug particles in combination with rapidly dispersing microgranules provide sufficiently hard, less friable tablets to afford packaging in high density polyethylene (HDPE) bottles and/or push-through or peel-off paper-backed blisters packaging for transportation, commercial distribution, and end use. In such cases, further coating with a non-polymeric compressible coating material (e.g., sucralose) may or may not result in a significant improvement in tableting properties.

In another embodiment, the present invention relates to a pharmaceutical dosage form as a rapidly dispersing tablet, comprising: (i) a first or second population of controlled-release particles, wherein each controlled-release particle comprises a core comprising at least one drug or a pharmaceutically acceptable salt, solvate, ester, and/or mixture thereof; disposing a delayed release coating comprising an enteric polymer over the core; and disposing a timed, pulsatile-release coating comprising an enteric polymer in combination with a water-insoluble polymer over the core; and an outer coating directly disposed over the timed, pulsatile-release coating comprising a compressible coating material (e.g., water-soluble sucralose) wherein said compressible coating material is substantially free of polymer and (ii) pharmaceutically acceptable excipients including fillers, diluents, compression aides and rapidly dispersing granules comprising a saccharide and/or sugar alcohol in combination with a disintegrant.

In yet another embodiment, the present invention relates to a method of preparing an orally disintegrating tablet comprising: (i) mixing one or more populations of controlled-release particles, as described herein, with rapidly dispersing granules comprising a saccharide and/or sugar alcohol in combination with a disintegrant, and other ODT excipients (e.g., a flavor, a sweetener, additional disintegrant, a compression-aid (a filler such as microcrystalline cellulose and/or spray dried mannitol), a colorant, etc), thereby forming a compression blend; and (ii) compressing the compression blend into an orally disintegrating tablet.

In another embodiment, the drug particles of the present invention are coated with a first coating with a water-insoluble polymer (e.g., ethylcellulose) by phase separation, a second coating in a fluid bed coater-with a water-insoluble polymer in combination with a gastrosoluble polymer at a ratio of from about 9:1 to about 5:5 as disclosed in U.S. Publication No. 2009/0202630, followed by a fluid-bed coating with compressible sucralose. In yet another embodiment of the present invention, the drug-containing particles are first coated with a film-forming water-insoluble polymer (e.g., ethylcellulose) by phase separation for a total weight gain of from about 30% to about 60% w/w in accordance with a co-pending U.S. Publication No. 2005/0232988,. The first coating is followed by a fluid-bed coating with compressible sucralose.

In certain other embodiments of the present invention, drug particles are taste masked by fluid-bed coating with a water-insoluble polymer (e.g., ethylcellulose with a mean viscosity of 10 cps when tested as a 5% solution in 80% toluene/20% alcohol at ambient temperature) in combination with a gastrosoluble pore-former such as Eudragit EPO, a cationic polymer, calcium carbonate or calcium succinate in accordance with the disclosures in U.S. Publication Nos. 2006/0078614, 2006/0105038, and 2006/0105039. Each of these taste-masked drug particles is further membrane coated with compressible sucralose.

In yet another embodiment of the present invention, the highly spherical drug particles having an coating of a water-insoluble polymer by temperature-induced phase separation with ethylcellulose with a mean viscosity of 100 cps when tested as a 5% solution in 80% toluene/20% alcohol at ambient temperature for a weight gain of from about 5% to about 20% w/w and an outer coating of water-insoluble polymer (e.g., ethylcellulose with a mean viscosity of 10 cps or higher) in combination with a reverse enteric polymer, is provided with an intermediate coating of a flavor-sweetener combination sandwiched between said first and second coatings in accordance with U.S. Publication No. 2009/0202630 in order to avoid experiencing the drug taste in case of accidental biting into coated drug particles. The triple-layer is further membrane coated with compressible sucralose.

The film-forming polymer applied on drug particles as a protective seal coating layer can comprise any water-soluble polymer. Non-limiting examples of suitable film-forming polymers include water-soluble, alcohol-soluble or acetone/water soluble hydroxypropyl methylcellulose (HPMC; e.g., Opadry® Clear from Colorcon), hydroxypropylcellulose (HPC; Klucel® LF from Aqualon), and polyvinylpyrrolidone (PVP). The amount of film-forming polymer applied on drug particles can range from about 0.5% to about 5%, including about 1 % to about 3%, or about 2% w/w.

Representative examples of water-insoluble polymers useful for taste-masking drug particles in accordance with the present invention include ethylcellulose, polyvinyl acetate (for example, Kollicoat SR#30D from BASF), cellulose acetate, cellulose acetate butyrate, neutral copolymers based on ethyl acrylate and methylmethacrylate, copolymers of acrylic and methacrylic acid esters with quaternary ammonium groups such as Eudragit NE, RS and RS30D, RL or RL30D and the like.

Representative examples of gastrosoluble organic or inorganic pore-forming agents useful for taste-masking drug particles in accordance with the present invention include, but are not limited to, calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, and the like and the mixtures thereof. The ratio of water-insoluble polymer to gastrosoluble organic or inorganic pore-former for producing taste-masked particles may typically vary from about 95/5 to about 50/50, or in some embodiments from about 85/15 to 65/35, at a thickness of from about 5% to about 50%, more particularly from about 10% to about 60% by weight of the coated drug particles.

In another embodiment, the pore-forming polymeric material consists essentially of a terpolymer based on aminoalkyl acrylate or methacrylate, butyl acrylate or methacrylate, and a methacrylate. In another embodiment, the pore-forming polymeric material may be a terpolymer based on dimethylaminoethyl methacrylate, butyl methacrylate, and methyl methacrylate; and in yet another embodiment, the terpolymer has an average molecular weight of 150,000 and the ratio of the monomers is 1:2:1 of methyl methacrylate, N,N-. dimethylaminoethyl methacrylate, and butyl methacrylate. An example of a pore-forming polymeric material is a polymer of the EUDRAGIT® E series (e.g., EUDRAGIT® EIOO or EUDRAGIT® EPO). A polymer of this series has a pKa of 6.3, is soluble in gastric fluid below pH 5 while it swells and/or is permeable in water and buffer solutions above pH 5.0. Saliva is typically in the pH range of about 6.7 to 7.4. Another example of gastrosoluble pore forming polymer is poly(vinylacetal diethylaminoacetate) e.g., AEA ® available from Sankyo Company Limited, Tokyo (Japan). In one embodiment, the reverse enteric polymer is a terpolymer based on dimethylaminoethyl methacrylate, butyl methacrylate, and methyl methacrylate. In another embodiment, the terpolymer has an average molecular weight of 150,000 and the ratio of the monomers is 1:2:1 of methyl methacrylate, N,N-dimethylaminoethyl methacrylate, and butyl methacrylate. The ratio of water-insoluble polymer to pore-forming polymeric material for producing taste-masked ranitidine HCl drug particles may typically vary from about 95/5 to about 50/50. The amount of the taste-masking coating ranges from about 5% to about 30%, by weight of the taste-masked ranitidine-containing granule, or about 5%-25%, about 5%-20%, about 5%-15%, about 5%-10%, about 10%-30%, about 10%-25%, about 10%-20%, about 10%-15%, about 15%-30%, about 50%-25%, about 15%-20%, about 20%-30%, about 20%-25%, or about 25%-30%.

The intermediate and the outer membranes described herein include one or more plasticizers. Representative examples of plasticizers that may be used to plasticize the membranes include glycerol and esters thereof preferably from the following subgroup: acetylated mono- or diglycerides (e.g., Myvacet® 9-45), glyceryl monostearate, glyceryl triacetate, glyceryl tributyrate, phthalates, preferably from the following subgroup: dibutyl phthalate, diethyl phthalate, dimethyl phthalate, dioctyl phthalate, citrates, preferably from the following subgroup: acetylcitric acid tributyl ester, acetylcitric acid triethyl ester, tributyl citrate, acetyltributyl citrate, triethyl citrate, glyceroltributyrate; sebacates, preferably from the following subgroup: diethyl sebacate, dibutyl sebacate, adipates, azelates, benzoates, chlorobutanol, polyethylene glycols, vegetable oils, fumarates, preferably diethyl fumarate, malates, preferably diethyl malate, oxalates, preferably diethyl oxalate, succinates, preferably dibutyl succinate, butyrates, cetyl alcohol esters, malonates, preferably diethyl malonate, castor oil (this being particularly preferred), and mixture. When used in an embodiment of the present invention, the plasticizer may constitute from about 3% to about 30% by weight of the water-insoluble polymer. In another embodiment, the plasticizer constitutes from 10% to about 25% by weight of the water-insoluble polymer. In still other embodiments, the amount of plasticizer relative to the weight of the water-insoluble polymer is about 3%, about 5%,∼o∼7%,∼o∼10%,∼o∼12%,∼o∼15%,∼o∼17%,∼o∼20%,∼o∼22%,∼o∼ 25%, about 27%, and about 30%, inclusive of all ranges and subranges therebetween. One of ordinary skill in the art would know to select the type of plasticizer based on the polymer or polymers and nature of the coating system (e.g., aqueous or solvent-based, solution or dispersion-based) and the total solids. Furthermore, in certain embodiments of the invention, the outer membrane containing reverse enteric polymer further comprises an anti-tack agent. Representative examples of anti-tack agents include talc, magnesium stearate and the like.

The orally disintegrating compositions of the present invention include rapidly dispersing granules comprising a disintegrant and a sugar alcohol and/or a saccharide. Non-limiting examples of suitable disintegrants for the disintegrant-containing granules can include disintegrants or so-called super-disintegrants, e.g. crospovidone (crosslinked PVP), sodium starch glycolate, crosslinked sodium carboxymethyl cellulose, low substituted hydroxypropylcellulose, and mixtures thereof. The amount of disintegrant in the rapidly dispersing granules can range from about 1%-10%, or about 5%-10% of the total weight of the rapidly dispersing granules, including all ranges and subranges therebetween.

Sugar alcohols are hydrogenated forms of carbohydrates in which the carbonyl group (i.e., aldehyde or ketone) has been reduced to a primary or secondary hydroxyl group. Non-limiting examples of suitable sugar alcohols for the rapidly dispersing granules of the orally disintegrating compositions of the present invention can include e.g. arabitol, isomalt, erythritol, glycerol, lactitol, mannitol, sorbitol, xylitol, maltitol, and mixtures thereof. The term "saccharide" is synonymous with the term "sugars" includes monosaccharides such as glucose, fructose, lactose, maltose, trehalose, and ribose; and disaccharides such as sucrose, lactose, maltose, and cellobiose. In one embodiment, non-limiting examples of suitable saccharides for use on the compositions of the present invention can include e.g. lactose, sucrose, maltose, and mixtures thereof. In another embodiment, the rapidly dispersing granules comprise at least one disintegrant in combination with a sugar alcohol. In another embodiment, the rapidly dispersing granules comprise at least one disintegrant in combination with a saccharide. In yet another embodiment, the disintegrant-containing granules comprise at least one disintegrant in combination with a sugar alcohol and a saccharide. The amount of sugar alcohol and/or saccharide in the rapidly dispersing granules ranges from about 99%-90%, or about 95%-90% of the total weight of the disintegrant-containing granules, including all ranges and subranges there-between. In one embodiment, the average particle size of a sugar alcohol and/or saccharide is 30 µm or less, for example about 1-30 µm, about 5-30 µm, about 5-25 µm, about 5-20 µm, about 5-15 µm, about 5-10 µm, about 10-30 µm, about 10-25 µm, about 10-20 µm, about 10-15 µm, about 15-30 µm, about 15-25 µm, about 15-20 µm, about 20-30 µm, about 20-25 µm, or about 25-30 µm.

The rapidly dispersing granules of the present invention can be prepared by any suitable method. For example, the rapidly dispersing granules can be prepared by granulation of one or more disintegrants and one or more sugar alcohols and/or saccharides in a high shear granulator, and dried in fluid bed equipment or on trays in a conventional oven to produce the rapidly dispersing granules, e.g. in the form of rapidly-dispersing micro granules. Rapidly dispersing microgranules can also be produced by the method described in U.S. Patent Application Publication No. 2005/0232988 A1.

The compositions of the present invention contain an amount of rapidly dispersing granules and/or the mixture of a disintegrant and a sugar alcohol and/or a saccharide sufficient to provide a suitable rate of disintegration in the oral cavity of a patient forming a smooth, palatable, easy-to-swallow suspension containing drug particles. The amount of a disintegrant in the rapidly dispersing granules and/or the amount of disintegrant-sugar alcohol/saccharide combination in relation to drug in the compositions of the present invention can be adjusted to provide a suitable disintegration rate, as well as to form a smooth, palatable, easy-to-swallow suspension containing drug particles. For example, the compositions of the present invention contain an amount of disintegrant-sugar alcohol/saccharide combination in relation to drug sufficient to provide an *in vitro* disintegration time of about :S 30 seconds (USP <701> Disintegration Test).

The amount of rapidly dispersing granules or the amount of rapidly dispersing granules (i.e., disintegrant-sugar alcohol/saccharide combination) in relation to taste-masked drug particles can vary depending upon the desired disintegration rate and the desired organoleptic properties including taste-masking, mouthfeel and aftertaste. The amount of rapidly dispersing granules in the compositions of the present invention can range from about 30% to about 90%, including about 40%, about 45%, about 50%, about 55%, about 60%, about 65%, about 70%, about 75%, about 80%, and about 85%, inclusive of all values, ranges, and subranges there-between. In one embodiment, the amount of rapidly dispersing granules is about 60-70% of the total weight of the composition. In another embodiment, the amount of rapidly dispersing granules is about 65% by weight.

The total amount of one or more multi-coated particle populations comprising a drug in the orally disintegrating compositions of the present invention can range from about 5% to about 50%, including about 5%, about 10%, about 15%, about 20%, about 25%, about 30%, about 35%, about 40%, about 45%, and about 50%, inclusive of all values, ranges, and subranges there between. In one embodiment, the amount of taste-masked drug particles in the orally disintegrating compositions of the present invention is about 30% by weight of the orally disintegrating composition.

In some embodiments, the ratio of population of taste-masked drug particles to the population of CR-coated particles in the ODT compositions of the present invention can vary from about 1 :4 to about 1: 1. The taste-masking layer(s) (as described herein) can be applied onto drug particles by any suitable combination of taste-masking methods, for example (1) coacervation followed by fluid bed coating, (2) fluid bed coating followed by coacervation, (3) coacervation followed by two successive fluid bed coating, and (4) fluid bed coating followed by coacervation followed by fluid bed coating.

The compositions of the present invention may further comprise one or more pharmaceutically acceptable, flavoring agents. Non-limiting examples of such flavoring agents include, for example, cherry, spearmint, orange, or other acceptable fruit flavors, or mixtures of cherry, spearmint, orange, and other acceptable fruit flavors, at up to about 5% based on the tablet weight. In addition, the compositions of the present inventions can also include one or more sweeteners such as aspartame, sucralose, or other pharmaceutically acceptable sweeteners, or mixtures of such sweeteners, at up to about 2% by weight, based on the tablet weight. Furthermore, the compositions of the present invention can include one or more FD&C colorants at up to about 0.2% to about 2% by weight, based on the tablet weight.

The compositions of the present invention can also include an additional disintegrant, in addition to the disintegrant in the disintegrant-containing granules (e.g., ranitidine containing and/or rapidly-dispersing granules). The additional disintegrant can be the same disintegrant used in the disintegrant-containing granules, or a different disintegrant. The additional disintegrant may be present in the compositions of the present invention at up to about 10% based on the tablet weight.

The compositions of the present invention can also include a pharmaceutically acceptable filler such as microcrystalline cellulose, e.g. Avicel PHI01, Avicel PHI02, Ceolus KG-802, Ceolus KG-1000, Ceolus UF 711, Prosolv SMCC 50 or SMCC 90 or other pharmaceutically acceptable grades of microcrystalline cellulose, as well as mixtures thereof. In one embodiment, the orally disintegrating compositions of the present invention comprise about 25-35% of drug particles coacervated with a taste-masking layer comprising a water-insoluble polymer (e.g., ethylcellulose) followed by a compressible coating layer of a non-polymeric sweetener, about 60-70% of rapidly-dispersing granules (e.g., comprising crospovidone and mannitol); about 5% of additional disintegrant (e.g., crospovidone); about 5% to 15% by weight of microcrystalline cellulose, about 0.5 - 2.0% of one or more flavors, and about 0.5%-1 % of a sweetener (e.g., sucralose). Furthermore, rapidly dispersing microgranules may be partly replaced by spray-dried mannitol or specially processed mannitol granules commercially available as "Parteck® 200 or 300" from Merck KGaA, "Ludiplus® (specially processed lactose granules)" and "Ludiflash® (specially processed mannitol granules) "from BASF, GalenIQ® agglomerated isomalt.
In accordance with certain embodiments of the present invention, the method may include the steps of:
i. taste-masking drug particles (e.g., crystals, drug-layered beads, granules, or pellets by controlled spheronization or powder layering using Granurex from Vector Freund (Iowa) with an average particle size of about 400 µm or less) by (a) solvent coacervation with a water-insoluble polymer (e.g., ethylcellulose with a mean viscosity of 100 cps) and optionally in combination with an inorganic or organic pore-former such as micronized calcium carbonate for a weight gain of from about 10% w/w to about 40% w/w or by (b) solvent coacervation with a water-insoluble polymer (e.g., ethylcellulose) for a weight gain of from about 10% w/w to about 20% w/w followed by fluid bed coating with a water-insoluble polymer (e.g., ethylcellulose) in combination with a gastrosoluble pore-former (e.g., calcium carbonate) in accordance with the disclosure in the co-pending US Patent Application Ser. Nos. 11/213,266 filed Aug. 26, 2005 (Publication No. U.S. 2006/0105038), 11/256,653 filed Aug. 26, 2005 (Publication No. U.S. 2006/0105039), or 11/248,596 filed Oct.12, 2005 (Publication No. U.S. 20060078614), and (c) applying a compressible coating with a non-polymeric compressible sweetener (e.g., sucralose), further coating the taste-masked drug particles with a compressible coating with non-polymeric compressible coating material such as sucralose;
ii. granulating a powder mixture of a sugar alcohol such as mannitol or a saccharide such as lactose and crospovidone, for example, using the disclosures in EP 0914818 and the co-pending US Patent Application Ser. No. 10/827,106 filed Apr. 19, 2004 (Publication No. U.S. 2005/0232988), to produce rapidly-dispersing micro granules;
iii. blending appropriate amounts of the taste-masked drug particles from step (i), rapidly-dispersing microgranules from step (ii) and other pharmaceutically acceptable excipients; and
iv: compressing the blend from step (iii) into orally disintegrating tablets in accordance with the disclosures of U. S. 6,964,779 and U.S. 5,700,492, comprising required doses of said drug which would rapidly disintegrate on contact with saliva in the buccal cavity forming a smooth, easy-to-swallow suspension and exhibit pharmacokinetics parameters in the range of 80.0% to 125.0: of 90% confidence interval.

One embodiment of a method for the preparation of a taste-masked composition of the present invention comprises:
a. a first coating step comprising coating in a fluid bed coater drug particles (e.g., crystals, drug-layered beads, granules, or pellets by controlled spheronization or powder layering using Granurex from Vector Freund (Iowa) with an average particle size of about 400 µm or less) with a water-insoluble polymer, an enteric polymer, or fatty acid and/or ester for a weight gain of 2% to 50% by weight;
b. a second optional coating step comprising coating said coated drug particles of step (a) with a second polymeric coating material comprising a water-insoluble polymer and an enteric polymer for a weight gain of 10% to 40% by weight to form controlled-release coated drug particles; and
c. an outer compressible coating with a non-polymeric sweetener (e.g., sucralose, lactitol, or maltitol) for a weight gain of 2% to 10% by weight to form compressible coated controlled release (CR) drug-containing microparticles.

The polymeric materials can comprise about 10% to about 30% by weight of said first or second membrane on said microcapsules.

In accordance with certain embodiments of the present invention, the method may include the steps of
i. drug-containing microparticles of a pharmacologically active drug or pharmaceutically acceptable salt, solvate, ester and/or mixture thereof, by coacervation with a water-insoluble polymer (e.g., ethylcellulose with a mean viscosity of 100 cps) for a weight gain of about 4-8% w/w;
ii. layering the low-dose component (e.g., an opioid analgesic such as hydrocodone) from a polymeric binder solution onto drug particles of step (i);
iii. applying a second taste-masking layer disposed over said opioid-coated drug particles comprising a hydrophilic polymer alone or in combination with a water-insoluble polymer in accordance with the disclosures of U.S. Patent Application Ser. Nos. 12/772,770 filed May 3, 2010 or 12/772,776 filed May 3, 2010; and
iv. applying an outer compressible coating with a non-polymeric sweetener (e.g., sucralose, lactitol, or maltitol) for a weight gain of 2% to 10% by weight to minimize membrane fracture during tableting of taste-masked drug particles.

In accordance with certain other embodiments of the present invention, the method may include the steps of:
i. preparing compressible coated CR drug containing particles with an average particle size of not more than 400 µm as described above;
ii. granulating a powder mixture of a sugar alcohol such as mannitol or a saccharide such as lactose and a super disintegrant such as crospovidone, to produce rapidly-dispersing microgranules;
iii. blending appropriate amounts of the CR-coated drug particles from step (i), rapidly-dispersing microgranules from step (ii) and/or other pharmaceutically acceptable excipients such as one or more flavoring agents, colorants, a sweetener, diluents/fillers/compression aides such as microcrystalline cellulose, spray dried lactose, and additional disintegrant; and
iv. compressing the blend from step (iii) into orally disintegrating tablets or rapidly dispersing tablets comprising required doses of the drug for oral administration in patients or subjects in need of medication. The ODT tablet thus produced would rapidly disintegrate on contact with saliva in the buccal cavity forming a smooth, easy-to-swallow suspension. The rapidly dispersing tablets rapidly disperse on contact with water or body fluid and exhibit desired pharmacokinetics parameters on digestion.

In one embodiment, the first coating step comprises (i) mixing a water-soluble polymer with a polar and/or nonpolar organic solvent mixture to dissolve said polymer and applying the coating onto said drug particles while maintaining said drug particles at a desired fluidized product bed temperature, and said second coating step involves (i) mixing said first coated drug particles with a first water-insoluble polymer (ethylcellulose) and a nonpolar organic solvent (cyclohexane) and a phase inducer (polyethylene wax) to form said drug particle-polymer mixture; (ii) heating said drug particle-polymer mixture at a first temperature so that said first polymeric material dissolves in said nonpolar organic solvent; (iii) cooling said drug particle - polymer mixture over time while stirring to a second temperature to form a dispersion of coated drug particles; (iv) recovering said coated drug particles; and (v) applying a compressible coating of a non-polymeric compressible material (e.g., sucralose) onto dried said coated drug particles. In another embodiment, an additional step of applying a flavor-sweetener coating composition onto said coacervated first polymer coated drug particles in fluid bed coater prior to applying the compressible coating may be included in the manufacturing process. In yet another embodiment, an additional step of applying a second coating composition onto said flavor coated drug particles in fluid bed coater prior to compressible coating may be included in the total manufacturing process.

In one embodiment, the drug particles with said first coating as described above are further coated with a second coating with steps comprising (i) mixing a water insoluble polymer (ethylcellulose), reverse enteric polymer (Eudragit EIOO), a plasticizer (triethyl citrate) with a nonpolar solvent to dissolve, (ii) homogeneously suspending an anti-tack agent (talc or magnesium stearate) and (iii) spraying onto singly coated drug particles while maintaining said singly coated drug particles at a desired product temperature and in an appropriately fluidized state to avoid agglomeration of said drug particles, and (iv) applying the compressible coating onto said coated drug particles. In another embodiment, an additional step of applying a flavor-sweetener coating composition onto said first polymer coated drug particles in fluid bed coater may be included in the total manufacturing process.

In another embodiment, both first and second coating steps applied in a fluid-bed coater involve the first membrane comprising a water-insoluble polymer and an optional water soluble or reverse enteric polymer applied for a gain of from about 2% to about 20% w/w and the second membrane applied for a total weight gain ofup to about 40% by weight of the coated drug particle comprising a water-insoluble polymer in combination with an enteric polymer at a ratio of from about 9:1 to about 1:4, followed by the compressible coating.

In another embodiment, the method of preparing the compositions of the present invention also includes a coating step to produce coated drug particles, i.e., sustained-release (SR), delayed-release (DR), timed pulsatile-release (TPR) and/or controlled-release (TPR-coating on SR-coating or DR-coating) beads, that are coated with a non-polymeric compressible coating material (e.g., sucralose). The taste-masked and/or controlled-release coated drug particles of the compositions of the present invention can be prepared by various methods, including solvent coacervation with a water-insoluble polymer such as ethylcellulose or a water-insoluble polymer in combination with a gastrosoluble pore-forming agent or fluid-bed coating with a water-insoluble polymer, an enteric polymer, a reverse enteric polymer, and a mixture thereof. The coating weight of the microencapsulated drug particles can range from about 5% to about 50% including about 10%, 15%, 20%, 25%, 30%, 35%, 40%, and 45%, inclusive of all ranges and subranges there-between.

Alternatively, the drug particles are first coated in a fluid-bed coater with a solution comprising a water-insoluble polymer (e.g., ethylcellulose) or an enteric polymer (e.g., hypromellose phthalate) and an organic solvent, and/or a solution comprising a water-insoluble polymer in combination with an enteric polymer for a weight gain of from about 10% to about 50% w/w. The ratio of water-insoluble polymer to enteric polymer can range from about 50/50 to 95/05, including about 55/45, about 60/40, about 65/35, about 70/30, about 75/25, about 80/20, about 85/15, and about 90/10, including all ranges and subranges there between. The coating weight of the micro encapsulated drug particles can range from about 5% to about 30% including about 10%, 15%, 20%, and 25%, inclusive of all ranges and subranges therebetween. Examples of such controlled-release coating processes are disclosed in US 6,627,223; US 6,500,454; US 7,387,793 and co-pending applications US 2006/0246134; US 2007/0190145; U.S.2007/0196491; U.S. 2009/0232885; U.S. 2009/0258066; WO 2010/096820; WO 2010/096814). These CR-coated drug particles are further coated with an outermost compressible coating comprising a non-polymeric compressible sweetener (e.g., lactitol).

One embodiment of a method for producing pleasant tasting orally disintegrating or rapidly dispersing tablet (i.e.,ODT or RDT) formulations of the present invention, comprising high drug load pellets produced using Granurex TM from Vector Corporation by controlled spheronization or powder layering or equivalent equipment (e.g., a rotogranulator), comprises
(i) charging an active pharmaceutical ingredient, an optional flow aid (Syloid, colloidal silica from W.R. Grace), and an optional binder (e.g., PVP K-30) into a Granurex bowl, (ii) spraying a 10% binder solution into the rotating material bed at a controlled rate while simultaneously adding the powder into the unit with a powder layer (K-Tron) at a controlled rate to bind the powder mix in the form of pellets, (iii) drying the pellets thus produced, (iv) applying one or more taste-masking membranes or CR coating membranes onto said pellets followed by applying the compressible coating of a non-polymeric compressible material, (v) preparing rapidly dispersing granules comprising a disintegrant, a sugar alcohol and/or a saccharide, and (vi) forming the oral dosage form. The step of forming the oral dosage form as an ODT or RDT can comprise, for example, compressing a blend comprising said compressible coated taste-masked or CR drug-containing microparticles and said rapidly dispersing granules and/or fillers, optionally with pharmaceutically acceptable flavorant(s), sweetener(s), other disintegrant(s), colorant(s) and/or compression aides such as microcrystalline cellulose in sufficient quantities into the orally disintegrating tablet or rapidly dispersing tablet form using a tablet press, such as a rotary tablet press equipped with an external lubrication system to lubricate the punches and dies prior to compression. These orally disintegrating tablets rapidly disintegrate upon exposure to the saliva in the mouth into a smooth, easy-to-swallow suspension with no gritty aftertaste. The rapidly dispersing tablets are suitable for oral administration in subjects or patients in need of medication for the treatment of a disease state by one of the modes of administration - (1) swallowing the whole tablet, (2) breaking the tablet into two halves for swallowing individually, and (3) dispersing the tablet in about 150 mL water, swirling, and drinking.

In another embodiment, the methods of the invention includes steps to prepare orally disintegrating tablets by mixing compressible coated, taste-masked or CR-coated microparticles, one or more flavoring agents, a sweetener, rapidly-dispersing microgranules, microcrystalline cellulose, additional disintegrant, and magnesium stearate and compressing this mixture into orally disintegrating tablets using a conventional rotary tablet press. The orally disintegrating tablets formed thereby may provide: rapid disintegration on contact with saliva in the buccal cavity, a pleasant taste (good creamy mouth feel), and rapid, substantially-complete release of the dose in the stomach or a desired target release or plasma concentration-time profile in patients for the treatment of one or more disease states.

In another embodiment, the methods of the invention includes steps to prepare rapidly dispersing tablets by mixing compressible coated, compressible coated, taste-masked or CR-coated microparticles, one or more fillers/diluents (e.g., spray dried lactose (e.g., Fast Flo Lactose), microcrystalline cellulose, spray dried mannitol, Ludiplus® (granulated lactose), Ludiflash® (granulated mannitol), Parteck® 200/300 (processed mannitol), additional disintegrant, and magnesium stearate and compressing this mixture into rapidly dispersing tablets using a conventional rotary tablet press. The rapidly dispersing tablets formed thereby would provide: rapid dispersion on contact with water or body fluids and rapid, substantially-complete release of the dose in the stomach or a desired target release profile.

Without being bound by the theory and/or mechanism of action, the compressible coating disposed directly on said coated drug particles (i.e taste-masked or controlled-release coated bitter drug cores) enable to accomplish- (i) minimize/eliminate membrane fracture during tableting of said compression blend into ODTs or RDTs, (ii) minimize/eliminate experiencing bitter drug taste on contact with saliva in the oral cavity, (iii) similar *in vitro* - *in* vivo release profiles from said controlled-release coated drug particles from said ODT or RDT formulations, and (iv) reduce in some cases, the coating levels required to achieve effective taste-masking in the absence of said compressible coating.

### In Vitro Disintegration Time/ Dissolution Testing

Disintegration times are measured using the USP <701> Disintegration Test procedures. The taste-masking properties of the taste-masked and/or CR-coated microparticles and the orally disintegrating tablets may be evaluated using a panel of healthy volunteers per approved protocol under supervision if needed. For example, the percentage of drug-release when tested for dissolution using USP Apparatus 2 (paddles @ 50 rpm) in 500 mL of saliva-simulating fluid at a pH of about 6.8-7 .0 (a release of not more than about 10% of the dose in about 3 minutes is considered acceptable) is determined. In addition, the rapid-release property in the stomach of the taste-masked microparticles and the orally disintegrating tablets may be evaluated by determining the percentage of drug-release when tested for dissolution using USP Apparatus 2 (paddles@ 50 rpm) in 900 mL of O.OIN HCl at 37.0±0.5°C (a release of not less than about 70% of the dose in about 30 minutes is considered acceptable in case of ranitidine hydrochloride). The potency of the tablets and the drug release profiles from CR-coated drug particles and ODT CR or RDT CR formulations are determined using United States Pharmacoepia Appratus 1 (baskets @ 100 rpm) or 2 (paddles @ 50 rpm) and a HPLC methodology, specifically developed for each drug.

In their various embodiments, the orally disintegrating tablet compositions of the present invention comprising compressible coated, taste-masked and/or CR microparticles exhibit one or more of the following properties:
(1) acceptable hardness and friability suitable for packaging in bottles and push-through or peel-off paper-backed blister packaging, storage, transportation and commercial distribution;
(2) disintegration on contact with saliva in the oral cavity in about 60 seconds forming a smooth, easy-to-swallow suspension with a pleasant taste (no grittiness or aftertaste), meeting the specification of not more than 30 seconds in the <USP 701> Disintegration Test;
(3) taste-masked and/or CR-coated drug particles exhibit smooth mouthfeel (non-gritty) and no aftertaste; and
(4) provide rapid, substantially-complete release of the dose from the taste-masked drug particles upon entry into the stomach, as evident by meeting the dissolution specifications of about 85% of the dose in about 45 minutes in 900 mL of 0.01N HCl buffer when tested for dissolution using USP Apparatus 2 (paddles@ 75 rpm) and/or provide desired (target) release profile(s) as evident by meeting the drug-release specifications when tested for dissolution using USP Apparatus 2 (paddles @ 50 rpm in 700 mL of 0.1N HCl for the first 2 hrs, followed by further testing in 900 mL buffer at pH 6.8).

In their various embodiments, the rapidly dispersing tablet compositions of the present invention comprising compressible coated, taste-masked and/or CR microparticles exhibit one or more of the following properties:
(5) acceptable hardness and friability suitable for packaging in bottles and push-through or peel-off paper-backed blister packaging, storage, transportation, commercial distribution, and end use;
(6) rapidly disperse on contact with water or body fluids thereby allowing oral administration in patients or subjects by one of three modes of administration for the treatment of one or more disease states;
(7) taste-masked and/or CR-coated drug particles exhibit resistance to membrane fracture during high speed compression into RDTs; and
(8) provide rapid, substantially-complete release of the dose from the taste-masked drug particles upon entry into the stomach, as evident by meeting the dissolution specifications of about 85% of the dose in about 45 minutes in 900 mL of 0.01 N HCl buffer when tested for dissolution using USP Apparatus 2 (paddles@ 75 rpm) and/or provide desired (target) release profile(s) as evident by meeting the drug-release specifications when tested for dissolution using USP Apparatus 2 (paddles @ 50 rpm in 700 mL of 0.1N HCl for the first 2 hrs, followed by further testing in 900 mL buffer at pH 6.8).

The compositions of the present invention are useful in treating or preventing disease conditions such as gastrointestinal disorders, cardiovascular diseases, central nervous system diseases, diabetes, Alzheimer or Parkinson's disease, schizophrenia, psychosis, epilepsy, depression, bipolar disorder, infection, obesity, or inflammatory disorders. The compositions of the present invention can comprise required amounts of the drug(s) in appropriate ratios providing desired plasma concentration - time profile depending on the severity of the disease state and and/or physical condition of the patient. For example, the compositions of the present invention can be administered in a single daily dose, or multiple daily doses, depending, for example, upon the severity of the condition and physical condition of the patient.

The following non-limiting examples illustrate the compositions of the present invention, comprising compressible coated, taste-masked and/or CR drug-containing microparticles or the orally disintegrating or rapidly dispersing tablet dosage forms, wherein the composition comprises a compressible coated drug, or a pharmaceutically acceptable salt, isomer, ester, and/or mixture thereof. The compositions of the present invention are prepared as described herein, and exhibit acceptable organoleptic or easy mode of oral administration and/or tableting properties and desired pharmacokinetics profiles upon ingestion depending on intended dosing regimens.

### EXAMPLE 1

1.A Microcapsules comprising Acetaminophen: Production of industrial scale acetaminophen microcapsules using an industrial scale 200-gallon, 500-gallon or 1000-gallon system uses a computerized recipe for the process (e.g., quantities for the 200-gallon system at 6% coating - Acetaminophen (APAP): 94.1 kg; Ethocel 100: 10.5 kg, Epolene: 2.1 kg and Cyclohexane: 142 gallons). The tank is heated to about 80°C through a pre-defined heating profile, under stirring at about 107 ± 5 rpm, followed by controlled cooling to ambient temperature, at NMT (not more than) 35°C. The microcapsule bed is subjected to vacuum filtration and rinsing with cyclohexane to wash off residual polyethylene. The microcapsules are transferred to the fluid bed dryer, subjected to a stepwise drying recipe (e.g., inlet temperature set at 25°C, 35°C and finally at 99°C), and dried for a period of 4-6 hrs to reduce the cyclohexane level to not more than 1000 ppm. The dried micro capsules are sieved through a 16 MG mesh screen to discard larger aggregates, if formed. Following the same procedure, a batch of microcapsules at 10% EC-I 00 coating was prepared by solvent coacervation in the 500 gallon system (single tank).
1.B Taste-masked Hydrocodone-layered Acetaminophen Microcapsules: Hydrocodone bitartrate (HCB) (240 g) was slowly added to an aqueous solution of hydroxypropyl cellulose (26.7 g of hydroxypropylcellulose (Klucel LF) in 2400 g water and mixed well to dissolve. Acetaminphen microcapsules at 6% by weight (3733.3 g) from step I .A, above were coated with the drug-layering formulation in a Glatt fluid-bed coater Glatt GPCG 5 (equipped with a 9" bottom-spray Wurster insert, 35 mm column height, 'D' air distribution plate, and 200 mesh product retention screen) under the following conditions - inlet air temperature: 60±3°C; product temperature: 40±5°C; atomization air pressure 2.0 bar; port size: 1.0 mm; flow rate: 8 mL/min increased in steps to 26 mL/min, air volume: 50 ± 5 CFM. Following the drug layering, the seal coating/taste-masking solution of hydroxypropylcellulose (210.5 g in 50/50 acetone/water at 10% solids, 1650 g each) was sprayed onto the drug layered beads for a weight gain of 5%. The dried IR drug particles were sieved through 35 and 80 mesh screens to discard oversized microparticles and fines.
1.C Compressible-coated Hydrocodone/ Acetaminophen Microcapsules: Hydrocodone/acetaminophen microcapsules (3400 g) from step 1.B, above were coated with a compressible coating formulation containing sucralose (179 g) dissolved in water (1014 g) for a weight gain of 5% in Glatt GPCG 5 equipped with a bottom spray Wurster insert at the processing conditions: - spray rate of 8 mL/min stepwise increased to 15 mL/min; product temperature of 32 ± 2°C. Upon completion of coating, the compressible coated drug particles are dried in the unit for about 5 min to minimize the residual moisture and sieved to discard fines and doubles if formed.
1.D Rapidly Dispersing Granules: The rapidly dispersing microgranules comprise a sugar alcohol such as mannitol and/or a saccharide such as lactose and a disintegrant such as crospovidone. The sugar alcohol and/or saccharide and disintegrant will typically be present in the rapidly dispersing microgranules at a ratio of from about 99:1 to about 90:10 (sugar alcohol and/or saccharide to disintegrant). For example, D-mannitol, a sugar alcohol with an average particle size of about 15 µm and Crospovidone XL-I 0, a super disintegrant, at a ratio of about 95/5 were granulated in a high shear granulator using purified water as the granulating fluid, wet milled, dried in a tray drying oven for an LOD of less than 1 % by weight, and dry milled to produce rapidly dispersing granules with an average particle size of about 200-400 µm.
1.E Hydrocodone / Acetaminophen ODTs: Microcrystalline cellulose (MCC), sucralose, the disintegrant, and the flavor (see table 1 for compositions of ODT formulations (e.g., Formula A to D) were blended in a V blender. This pre-blend, required amounts of taste-masked hydrocodone/acetaminophen microcapsules of step 1.C and I .A (acetaminophen at 10% EC-I 00 coating), and rapidly dispersing micro granules from step 1.D, above were blended in a V blender for 30 minutes to achieve blend uniformity, and compressed into 500 mg acetaminophen OTDs weighing about 1400 mg. While Formulas A, B, and D were compressed on Hata Tablet Press equipped with Matsui Ex-Lub System, an external lubrication device for lubricating punch and die surfaces just prior to each compression cycle, the compression mix C was blended with sodium stearyl fumarate prior to compression on the Hata press. Formulas A to D consist 5-mg, 5-mg, 25-mg and 34-mg hydrocodone bitartrate, respectively. As shown in Table 1, as the percent of compressible coated hydrocodone/acetaminophen microcapsules increased, replacing the hydrocodone/acetaminophen microcapsules coated with a polymeric compressible coating material (e.g., hydroxypropylcellulose (Klucel LF)) and/or the acetaminophen microcapsules having no compressible coating layer disposed over the hydrophobic taste-masking layer, the tableting properties improved, i.e., tablet hardness increased while the friability decreased, and tablets with improved tableting could be compressed even at lower compression forces, as to be anticipated.

**Table 1: Hydrocodone Bitartrate/Acetaminophen ODTs**

| Ingredient (%) | **Formula D (34/500-mg)** | **Formula C (25/500-mg)** | **Formula B (5/500-mg)** | **Formula A (5/500-mg)** |
|---|---|---|---|---|
| 5% Klucel/HCB/APAP (6%) | | | | **7.97** |
| Sucralose/Klucel/HCB/APAP (6%) | 45.31 | 32.95 | 6.59 | |
| APAP (PE378; 10%) | | 10.86 | 34.03 | |
| Rapidly Dispersing Granules | 31.64 | 33.14 | 32.19 | 30.15 |
| MCC (Avicel PH101) | 10.00 | 10.00 | 10.00 | 10.00 |
| Mannitol (Parteck M200) | 5.00 | 5.00 | 10.00 | |
| Crospovidone (XL-10) | 5.00 | 5.00 | | 5.00 |
| Croscarmellose Na (Ac-Di-Sol) | | | 3.00 | |
| Sucralose | 1.80 | 1.80 | 1.70 | 1.80 |
| Cherry Flavor | 1.25 | 1.25 | 1.50 | 1.25 |
| Magnesium stearate | Traces | Traces | | |
| Sodium stearyl fumarate | | | 1.00 | |
| Compression Force (kN) | At 12.5 kN | At 13.0 kN | At 20 kN | At 20 kN |
| Tablet Weight (mg) | 1410 mg | 1414 mg | 1402 mg | 1402 mg |
| Thickness (mm) | 6.40 mm | 6.33 mm | 6.17 mm | 6.37 mm |
| Hardness (N) | 72 N | 71 N | 60.8 N | 53.1 N |
| Friability (%) | 0.12% | 0.11% | 0.05% | 0.35% |
| Compression Force (kN) | At 16 kN | At 15 kN | At 24 kN | At 24 kN |
| Tablet Weight (mg) | 1412 mg | 1412 mg | 1402 mg | 1402 mg |
| Thickness (mm) | 6.17 mm | 6.26 mm | 5.95 mm | 6.17 mm |
| Hardness (N) | 107 N | 79 N | 80 N | 71.1 N |
| Friability (%) | 0.08% | 0.03% | 0.15% | 0.18% |

### EXAMPLE 2

2.A Microencapsulation in 5-Gallon Solvent System: Ranitidine hydrochloride (Form II from Shasun Drugs and Chemicals meeting desired particle size/aspect ratio specifications) was fluid-bed coated with an aqueous solution of Opadry Clear (hypromellose) at 4% solds in Glatt GPCG 1 (VersaGlatt) equipped with top spray for a weight gain of 2% w/w. Spraying conditions - Port size: 0.8 mm; atomization air pressure: 1.5 bar; bottom air distribution plate: granulation plate; inlet air temperature: 60°C; product temperature: 28-34°C; spray rate: 5 mL/min, Outlet flap: 40%; and shake interval/time: 30 sec/3 sec. The fluid-bed coated ranitidine was taste-masked by solvent coacervation in a 5-gallon system. The 5-gallon system filled with 10,000 g of cyclohexane was charged with ethylcellulose (Ethocel Standard Premium 100 from Dow Chemicals; 200 g), polyethylene (Epolene C-1 O; 200 g), and the drug (466.7 g). The system was subjected to a controlled heating cycle to achieve a temperature of 80°C to dissolve ethylcellulose while agitating the contents at a speed of 300 RPM. Thereafter the system was subjected to a cooling cycle to <30°C in not less than 45 min to wrap the drug crystals with a smooth coating at 30% by weight and avoiding formation of agglomerates. The microcapsules were separated by decanting, washed with fresh cyclohexane, and dried in the hood. The microcapsules sieved through 30 mesh (< 590 µm) screen were collected. One batch of fluid-bed coated ranitidine was also microencapsulated with Ethocel and micronized calcium carbonate, an inorganic gastrosoluble pore-former at a ratio of 8:3 by homogeneously dispersing the slurry containing the micronized pore former at the tank temperature of about 58°C during the cooling cycle.
2.B Compressible Coated Ranitidine Microcapsules: Ranitidine microcapsules (EC-100 coated, 3401 g) from step 2.A, above are coated with a compressible coating formulation containing sucralose (179 g) dissolved in water (1014 g) for a weight gain of 5% in Glatt GPCG 5 following the procedures described in step 1.C. Ranitidine microcapsules (EC-100/Ca C03 coated, 3401 g) from step 2.A, above are also coated with a compressible coating formulation containing sucralose (179 g) dissolved in water (1014 g) for a weight gain of 5% in Glatt GPCG 5.
2.C ODTs Comprising Compressible Coated Ranitidine Microcapsules: Taste-masked ranitidine drug particles (24-33% w/w of EC-100 and EC-100/CaC03) coated from step 2.A, above, rapidly dispersing granules (68-55% w/w), a disintegrant (5% w/w), sweetener (0.1 - 0.5% w/w), flavor combination (0.5-3.5% w/w), MCC (0-10% w/w), and colorants (0.1 - 0.3% w/w) were blended together in a V blender and compressed into 150 mg (as ranitidine base) ODTs. These tablets had acceptable organoleptic properties and dissolution profiles. The compressible coated microcapsules (EC-I 00 or EC-I OO/CaC03 coated) are also compressed into ODTs.

**Table 2: Compositions and Properties of ODTs of Ranitidine HCl**

| | Orally Disintegrating Tablets - % per Tablet | | | |
|---|---|---|---|---|
| | Formula A | Formula B | Formula C | Formula D |
| Microcapsules (EC-100) (Formula 1) | 24.00 | | | |
| Compressible Coated (CC)/EC-100 (Formula 2) | | | 33.00 | |
| Microcapsules (EC-100/CaCO₃) (Formula 3) | | 25.26 | | |
| CC Microcapsules (EC-100/CaCO₃) (Formula 4) | | | | 34.74 |
| RD Granules | 67.40 | 66.14 | 58.40 | 56.66 |
| Crospovidone XL-10 | 5.00 | 5.00 | 5.00 | 5.00 |
| Sucralose | 0.20 | 0.20 | 0.20 | 0.20 |
| Vanilla Mint Combo | 3.25 | 3.25 | 3.25 | 3.25 |
| Red/Yellow/Blue, | 0.15 | 0.15 | 0.15 | 0.15 |
| Magnesium stearate | Traces | Traces | Traces | Traces |
| | | | | |
| Compression Force (kN) | 32.0 | | 30.0 | |
| Tablet Weight (mg) | 1025.6 | | 967.1 | |
| Thickness (mm) | 5.49 | | 5.18 | |
| Hardness (N) | 71 | | 71 | |
| Friability (%) | 1.99 | | 0.80 | |
| Dissolution at 30 min (%) | 84 | | 94 | |

### EXAMPLE 3

3.A Diphenhydramine HCl IR Beads: A grounded stainless steel tank equipped with a propeller mixer was filled with 300 kg of Acetone NF. Purified Water USP (93.3 kg) was slowly added to the tank while stirring the tank at approximately 850 rpm± 25 rpm. Diphenhydramine hydrochloride (76.5 kg) was slowly added into the tank to dissolve, followed by adding 8.42 kg of Klucel LF into the same tank to dissolve while constantly stirring. Hydroxypropylcellulose (Klucel LF; 6.12 kg) was slowly added into a separate stainless steel tank containing 86.4 kg of acetone and 9.6 kg of water to dissolve. 60-80 mesh sugar spheres (215 kg) were charged into a preheated Glatt fluid-bed coater, GPCG 120, equipped with a 32" bottom spray Wurster insert (three 23.75" high; inner bottom air distribution plate: G 1; outer plate: C 1; product retention plate: 100 mesh screen; nozzle tip port size: 50 mm; process air temperature: 70°C; process air volume: 1500 CFM; spray rate: 1500 (range: 200-2000) g/min; product temperature: 49-51 °C). The batch recipe would proceed automatically with the drug layering step at 300 g/min and increase flow rates and inlet temperatures accordingly. Processing parameters were recorded approximately every 30 minutes (minimum). The product was periodically inspected through the sample port to ensure that aggregation does not occur during spraying. Once the drug-layering solution was sprayed, the seal coating was applied at a spray rate of 300 g/min for a 2% weight gain. Following completion of seal coating, the beads were dried in the unit to drive off excess of residual acetone. The IR beads thus produced were sieved through #32 and # 80 mesh screens to discard over and under sized beads.
3.B Taste-masking of Diphenhydramine HCl IR Beads: The twin tank 500-gallon coacervation system with each tank was charged with 415 gallons of cyclohexane, 61.5 kg of IR beads of diphenhydramine hydrochloride, 20.5-25.1 kg of ethylcellulose and 10-25 kg of polyethylene while stirring at 75±5 rpm. The system was subjected to a computer controlled 'heat and hold' cycle whereby the contents of the tank were heated to about 80°C to completely dissolve ethylcellulose, and thereafter to a 'filter and fluid-bed dry' routine whereby the contents of the tank were cooled to about 30°C. As the temperature goes below about 65°C, the ethylcellulose which is no longer soluble in cyclohexane starts precipitating out. While so doing, being assisted by the phase inducer, polyethylene, it coats individual drug particles to provide taste-masking. Upon cooling to ambient temperature, the microcapsules thus formed were vacuum-filtered, rinsed with fresh cyclohexane, and vacuum dried in the fluid bed equipment to achieve pre-determined residual solvent level. The dried microcapsules were sieved through 40 mesh sieve using a Kason siever and discharged into fiber drums double-lined with polyethylene bags. The microcapsules thus obtained had an assay of approximately 18 .4-19 .4 %, exhibited a particle size of not more than 10% retained on 40 mesh sieve and not more than 10% passing through 80 mesh sieve, and a mean dissolution of aboutl 1-22% in 5 minutes and about 62-70% in 45 minutes, when dissolution tested in water at 7 5 rpm.
3.C Lactitol coating of Diphenhydramine Microparticles: Diphenhydramine microparticles from step 3 .B, above are coated with a compressible coating formulation containing hydroxypropylcellulose (Klucel LF; g) and lactitol (g) dissolved in water (% solids) for a weight gain of 5% in a Glatt fluid-bed coater, Glatt GPCG 3 following the procedures disclosed in step 1.C, above. The dried compressible coated beads are sieved through 50 and 80 mesh screens to discard oversized microparticles and fines.
3.D ODTs Comprising Diphenhydramine Microcapsules: Diphenhydramine microparticles (34-40% w/w) from step 3.B, above or compressible coated diphenhydramine microparticles (32-37 % w/w) from step 3.C, above are blended with rapidly dispersing microgranules (50-56% w/w) and a pre-blend mix comprising crospovidone (5% w/w), microcrystalline cellulose (4-10% w/w), sucralose (0.2-0.5%), vanilla mint flavor (0.4-1.0%), and colorants (0.1-0.3%) in a V-blender for 15 min and compressed into ODTs using magnesium stearate as an external lubricant. These tablets exhibit acceptable organoleptic properties, tableting, and drug release profiles.

### EXAMPLE 4

4.A Dicyclomine HCl IR Beads: Povidone (PVP K30; 100.0 g) was slowly added to 75/25 95% ethanol/water (2325.0 g of 95% ethanol and 775.0 g of water) until dissolved under constant stirring for not less than 10 min. Dicyclomine hydrochloride (800.0 g) was slowly added while stirring, until dissolved. A Glatt GPCG 3 equipped with a 7" bottom spray Wurster 8" high column, partition column gap of 15 mm from the 'B' bottom air distribution plate covered with a 200 mesh product retention screen (0.8 mm port nozzle) was charged with 2800 g of 60-80 mesh sugar spheres and sprayed with the dicyclomine solution (20% solids) at an initial rate of 5 g/min with a stepwise increase to 15.5 g/min, at an inlet air volume of 90-105 m3 /hr, air atomization pressure of 1.50 bar while maintaining the product temperature of 37±3°C. Following rinsing of the spray system with 50 g of ethanol, the drug-layered beads were dried in the Glatt unit for 50 min to drive off residual solvents (including moisture). The resulting dicyclomine IR beads were sieved through 35 and 120 mesh screens to discard oversized particles and fines. A batch of dicycloamine IR beads with a drug load of 30% w/w is also prepared using Cellets 200 instead of Cellets 100 (microcrystalline cellulose spheres).
4.B Dicyclomine HCl SR Beads: Ethylcellulose (EC-10, Ethocel Premium 10 from Dow Chemicals; 159.1 g) was slowly added to 95% ethanol while stirring constantly until dissolved. Triethyl citrate (TEC; 15.9 g) was slowly added until dissolved. A Glatt GPCG 1 equipped with a 6" bottom spray Wurster 6" high column, 'B" bottom air distribution plate covered with a 200 mesh product retention screen, 0.8 mm port nozzle, was charged with 700 g of IR beads from step 4.A, above. The IR beads were sprayed with the SR functional polymer coating formulation (10% solids) at a product temperature of 33±3°C, atomization air pressure of 1.50 bar, inlet air flow of 50-75 m3 /hr, and an initial flow rate of 1 g/min with a stepwise increase to 6 g/min for a SR coating weight of 20%. Following spraying, the coated beads were dried in the Glatt unit for 30 min to drive off residual solvents (including moisture). The resulting SR beads were sieved to provide particles having a mean particle size of less than about 500 µm. A batch of 15% SR (EC-10/TEC) coated dicycloamine beads is also prepared using IR beads (30% drug load on Cellets 200).
4.C Dicyclomine HCl CR Beads: Ethylcellulose (EC-10; 93.0 g) was slowly added to acetone/water at 90/10 (1876.4 g of acetone and 208.5 g of water) while stirring rigorously to dissolve. Hypromellose phthalate (HP-55 from Shin Etsu Chemical Company; 50.7 g) was added to the EC-10 solution while stirring vigorously until dissolved. TEC (25 .4 g) was added to the solution until dissolved/dispersed homogeneously, thereby forming a TPR coating formulation. The SR beads at 15% coating (395 g) prepared in Example 4.B were fluid-bed coated with the TPR coating formulation (7.5% solids) in a Glatt 1 equipped with a 4" Wurster insert at a product temperature of 33±2°C, atomization air pressure of 1.50 bar, inlet air volume of 70-90 m3 /h, and a spray flow rate of 3-6 g/min for a TPR coating level of 30% by weight. Samples were pulled at a coating level of 15%, 20% and 25% by weight for drug release testing. Dried beads with a mean particle size of less than about 355 µm were collected by sieving. The 15% SR (EC-10/TEC) coated dicycloamine beads (Cellets 200) are also coated with the TPR (EC-I O/HP-55/TEC at 60/30/10) formulation for a weight gain of 20% w/w to produce CR beads (Cellts 200).
4.D Compressible Coating of Dicyclomine CR Beads: The dicycloamine SR beads at 20% coating from step 4.B, above are also coated with the compressible coating formulation containing maltitol for a weight gain of 5% w/w, following the procedures disclosed in step 1.C, above. Following the same procedure, the dicycloamine CR beads from step 4.C, above are also coated with the compressible coating formulation containing sucralose for a weight gain of 5% w/w.
4.E Dicyclomine HCl ODT CR: Pharmaceutically acceptable ingredients (i.e., cherry flavor (0.5-1.5% w/w), sucralose (0.1 -0.5% w/w), crospovidone (Crospovidone XL-10; 3-5% w/w), and microcrystalline cellulose (Ceolus KG-802; 3-10% w/w), are first blended in a V blender to achieve a homogeneously blended pre-mix. The rapidly dispersing microgranules (prepared as described in Example 1.D, above; 54-65% w/w) are blended with the compressible coated dicyclomine HCl CR or SR beads (22-33% w/w) from step 4.D, above and the pre-mix previously prepared in a twin shell V-blender, and compressed into 40-mg dicyclomine HCl ODT CR or ODT SR.

Thus, the orally disintegrating tablets (e.g., ODT SR or ODT CR) are compressed using a production scale Hata tablet press equipped with an external lubrication system (Matsui Ex-Lub System) under tableting conditions optimized exhibit acceptable tableting properties suitable for packaging in HDPE bottles, Aclar 200 blisters with a peel-off paper backing, and/or 'push-through' Aclar blister packs. For example, ODTs comprising 40 mg dicyclomine HCl as compressible coated SR or CR beads are compressed at the following conditions: - tooling: 14 mm round, flat face, radius edge; compression force: 12-16 kN; mean weight: 800 mg; mean hardness:- 30-60 N; and friability: 0.2-0.4%. Dicyclomine HCl ODT SR or ODT CR tablets thus produced would rapidly disintegrate in the oral cavity creating a smooth, easy-to-swallow suspension comprising coated dicyclomine HCl beads, having a release profile suitable for a once- or twice-daily dosing regimen.

### EXAMPLE 5

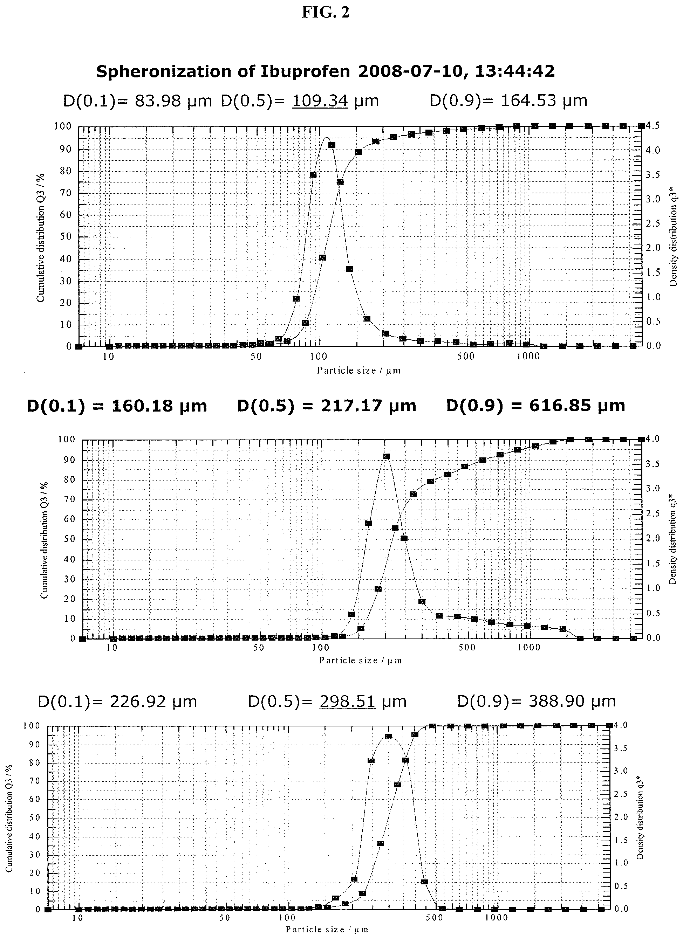

5.A Ibuprofen Pellets by Controlled Spheronization: Povidone (PVP K-30; 50 g) was slowly added to purified water (500 g) while constantly stirring to prepare a polymer binder solution at 10% w/w solids. Ibuprofen (2000 g) blended with 10 g of colloidal silica (a flow aid, Syloid from W.R. Grace) was charged into the product bowl of Granurex GX-40 from Vector Corporation (Iowa, USA). A 10% PVP binder solution was sprayed into the rotating material bed at a controlled rate while simultaneously the powder is added into the unit with a powder layer (K-Tron) at a controlled rate. Optimized parameters - inlet temperature: 50°C; roller speed: 300 RPM; slit air volume: 10 CFM; processing time: 20 min. Following completion, the pellets were dried in the unit. The pellets (batch size: 2 kg with a usable yield of about 96%) thus prepared had an average particle size of 109 µm (see FIG. 2.A for particle size distribution profile determined by Sympatec Laser Particle Sizer. Following a similar procedure (except that povidone (50 g) was blended with ibuprofen and Syloid and a faster spray rate was employed), two batches of pellets with an average particle size of 217 and 198 µm were also prepared (see FIG. 2.B for particle size distribution profile).
5.B Ibuprofen Pellets by Powder Layering: One set of trials utilized powder layering of Ibuprofen onto 50-70 mesh lactose/starch spheres in Granurex GX-40. The povidone solution (at 10% solids) was sprayed at a spray rate of 12-16 g/min and powder spray rate. The ibuprofen pellets thus prepared had a drug load of about 34% by weight and an average particle size of about 300 µm (see FIG. 2.C for laser particle size data).
5.C Niacin Pellets by Controlled Spheronization: Niacin (2000 g nicotinic acid from Lonza) with an average particle size of about 12 µm as determined by Malvern Particle Sizer, is blended with colloidal silica (10 g Cab-0-Sil 10 from Cabbott Corporation) and Povidone (50 g PVP K-30). Niacin pellets with an average particle size of 300 µm are prepared by Controlled Spheronization using Granurex GX-35 as disclosed in Step 5.A, above.
5.D Niacin SR Pellets: The niacin pellets from step 5.C, above (1400 g) is first provided with a seal coat with Opadry Clear at about 2% by weight in Glatt GPCG 3, equipped with a 7" bottom spray Wurster insert, 7 13/16" partition column, 'C' air distribution plate covered with a 200 mesh product retention screen, and 16 mm tubing. Following the procedures disclosed in step 4.B above, the seal-coated pellets are SR coated with a solution ofEC-10 (Ethocel Standard Premium 10 cps; 230 g) and triethyl citrate (TEC) (25.6 g) dissolved in acetone (2180.3 g) - water (384.7 g) mixture (6% solids) for a weight gain of 20% in the same Glatt unit at the following process parameters - inlet temperature: 45-48°C; inlet air volume: 40-45 cfm; flow rate: 8 mL/min to 18 mL/min (stepwise increase); atomization air pressure: 1.25 bar; nozzle port diameter: 1.0 mm; product temperature: 35-400C. Samples are pulled at a coating level of 10%, 15%, and 17.5% for testing for drug release.
5.E Compressible Coating of Niacin SR Pellets: Niacin SR pellets at 5% w/w coating (1000 g) are further coated with sucralose at about 20% w/w coating following the procedures of step 1.C above.
5.F Rapidly Dispersing Tablets Comprising Niacin Pellets: 75-85 parts of Compressible-coated Niacin SR pellets from step 5.E, above, 10-15 parts of microcrystalline cellulose, 0-10 parts rapidly dispersing granules, and 1.5-2.5 parts of crospovidone are first blended in a V blender for 20 minutes, and then 0.5 part of sodium stearyl fumarate is added to the blender to homogeneously distribute the lubricant by blending for about 10 minutes. The compression blend thus produced is compressed into 500 or 1,000 mg RDTs (rapidly dispersing tablets) using a rotary tablet press.
6.A Melperone HCl IR Beads: Melperone HCl (15.0 kg) was slowly added to 50/50 acetone/water (3425 g each) while stirring to dissolve. Glatt GPCG 120 equipped with an 18" bottom spray Wurster 23.75"column, 50 mm partition gap, 16 mm tubing, outer 'G' and inner "C" bottom air distribution plate covered with a 100 mesh product retention screen, and 3.0 mm nozzle with HS Collar at atomization air pressure of 2.5 bar., was charged with 43.8 kg of 45-60 mesh sugar spheres that were sprayed on at 100 g/min with a stepwise increase to 700 g/min while maintaining the target fluidization air volume at 500-900 CFM and product temperature at 32°C (range: 29-36°C. A seal coat of Klucel LF dissolved in acetone/water was applied at 2% by weight and the IR beads were dried to drive away moisture and acetone. The IR beads were sieved to discard oversized(> 500 µm or 35 mesh) beads and fines (< 80 mesh).
6.B Dibasic sodium Phosphate Anhydrous (DPA) Buffer Layering: Anhydrous disodium phosphate (6.2 kg) is added to purified water under stirring until dissolved. The buffer solution was sprayed onto the IR beads (52.6 kg) at a fluidization air volume of 650 (400-800) CFM. After optionally rinsing the buffer coated beads with a solvent, a seal coat of Klucel LF for a gain of about 2% by weight was applied. The dried IR beads were dried for 5 min to drive off residual solvents and sieved (e.g., using 35 and 80 mesh sieves) to discard oversized beads and fines.
6.C Melperone HCl SR Beads: The buffer-coated beads from Example 6.B (34.0 kg) were coated in the fluidized bed coating apparatus with an SR coating of plasticized (e.g., triethylcitrate at 10% w/w of ethylcellulose) water-insoluble polymer (e.g., 13 .9 kg ethyl cellulose and 1.1 kg dibutyl sebacate). A compressible coating solution (e.g., hydroxypropylcellulose such as Klucel® LF) dissolved in a solvent is sprayed onto the buffer coated beads for a weight gain of about 2% by weight. The resulting SR beads are further coated with a compressible coating of sucralose for a weight gain of 4% and dried to drive off residual solvents.
6.D Controlled-Release ODT Containing SR Beads: Rapidly dispersing microgranules (4295 g) are blended with compressible coated SR beads (3720 g) and the pre-blend containing other pharmaceutical acceptable ingredients (e.g., peppermint flavor: 100 g, sweetener (sucralose): 35 g, crospovidone: 500 g, silicon dioxide (Cab-0-Sil): 25 g, and microcrystalline cellulose (Avicel PHIO): 1350 g) in a twin shell V-blender for a sufficient time to obtain a homogeneously distributed blend for compression. ODTs comprising 50 mg melperone HCl as compressible coated SR Beads are compressed using a production scale tablet press (Hata) equipped with an external lubrication system at the following conditions: - tooling: 15 mm round, flat face, radius edge; compression force: 16 kN; mean weight: 1000 mg; mean hardness (target): 45 N; and friability: < 0.5%. The resulting ODT (50 mg dose) thus produced rapidly disintegrates in the oral cavity, creating a smooth, easy-to-swallow suspension comprising coated beads and provides an expected a drug-release profile suitable for a once-daily dosing regimen.

## Claims

1. A pharmaceutical composition comprising compressible coated microparticles comprising:
a. drug particles comprising a drug and/or a pharmaceutically acceptable salt, ester, polymorph, or solvate thereof;
b. at least one modified-release membrane layer comprising a water insoluble polymeric material disposed over the drug-containing cores; and
c. a compressible coating layer comprising a non-polymeric sweetener, disposed directly over the modified release coated drug particles from (b).

2. The pharmaceutical composition of claim 1, wherein said modified-release membrane layer is applied for a weight gain of from 5% to 50% by weight based on the total weight of the compressible-coated microparticles.

3. The pharmaceutical composition of claim 1, wherein said modified-release membrane is a taste-masking layer, wherein said taste-masking layer is applied for a weight gain of from 5% to 50% by weight based on the total weight of the compressible-coated microparticles.

4. The pharmaceutical composition of claim 3, wherein said taste-masking layer further comprises a gastrosoluble pore-former, wherein the ratio of water-insoluble polymeric material to gastrosoluble pore-former in said layer varies from 95:5 to 50:50 and the total layer is applied for a weight gain of from 5% to 50% by weight based on the total weight of the compressible-coated microparticles and wherein said taste-masking layer effectively masks the taste of the drug and/or pharmaceutically acceptable salt, ester, or solvate thereof and provides substantially complete release of the dose upon entry into the stomach.

5. The pharmaceutical composition of claim 3, further comprising a sealant layer comprising a hydrophilic polymer disposed over said drug-containing core particle, wherein said sealant layer is applied for a weight gain of from 1% to 10% by weight based on the total weight of the compressible-coated microparticles.

6. The pharmaceutical composition of claim 3, further comprising a second membrane layer comprising a water-insoluble polymer in combination with a gastrosoluble pore-forming polymer disposed over said taste-masking layer, wherein said second membrane layer is applied for a weight gain of from 5% to 50% by weight based on the total weight of the compressible-coated microparticles, and wherein said first and/or second coating(s) substantially masks the taste of drug- containing core particle.

7. The pharmaceutical composition of claim 3, wherein said pharmaceutical composition releases at least 70% of said drug or a pharmaceutically acceptable salt, ester, solvate, or combination thereof, upon entering the stomach or within 30 minutes when tested for dissolution in simulated gastric fluid or 0.01 N HC1 in United States Pharmacopoeia Apparatus 2 (paddles at 50 rpm in 900 mL of pH 1.2 or pH 2.0 buffer).

8. The pharmaceutical composition of claim 1, further comprising a sealant layer comprising a hydrophilic polymer disposed over said drug-containing core particle.

9. The pharmaceutical composition of claim 1, wherein said modified-release coated membrane layer is applied for a weight gain of from 5% to 25% by weight based on the total weight of the compressible-coated microparticles.

10. The pharmaceutical composition of claim 9, wherein said pharmaceutical composition provides a sustained-release profile of said drug or a pharmaceutically acceptable salt, ester, solvate, or combination thereof, upon entry into the gastrointestinal tract or when tested for dissolution in United States Pharmacopoeia Apparatus 2 (paddles at 50 rpm at 37°C in 700 mL of pH 1.2 buffer for first 2 hrs, followed by further testing at pH 6.8 obtained by the addition of 200 mL of a pH modifier).

11. The pharmaceutical composition of claim 9, wherein said membrane layer further comprises an enteric polymer wherein the ratio of water-insoluble polymeric material to enteric polymer in said first membrane varies from 9:1 to 1:2 and the total membrane is applied for a weight gain of from 10% to 40% by weight based on the total weight of the compressible-coated microparticles.

12. The pharmaceutical composition of claim 9, further comprising a second membrane, wherein the second membrane comprises a water-insoluble polymer in combination with an enteric polymer, wherein the ratio of water-insoluble polymer to enteric polymer in said second membrane varies from 9:1 to 1:2 and the total membrane is applied for a weight gain of from 10% to 40% by weight based on the total weight of the compressible-coated microparticles.

13. The pharmaceutical composition of claim 1, wherein said modified-release coated drug microparticles have a mean particle size of 500 µm or less.

14. An orally disintegrating tablet (ODT) or a rapidly dispersing tablet (RDT) composition comprising a taste-masked and/or modified-release coated drug- containing cores of claim 1 or 9 comprising:
a. drug particles comprising a therapeutically effective amount of a drug and/or a pharmaceutically acceptable salt, ester, or solvate thereof;
b. one or more polymeric membranes, each membrane comprising a water-insoluble polymer or optionally a water-insoluble and gastrosoluble polymeric blend material;
c. a compressible coating with a non-polymeric sweetener selected from the group consisting of sucralose, lactitol, sorbitol, maltitol, or a mixture thereof, directly disposed over modified release coated drug particles of step (b.); and
d. rapidly dispersing granules comprising (i) a disintegrant and (ii) a sugar alcohol, a saccharide, or a mixture thereof, at a ratio of from 90:10 to 99:1.

15. The orally disintegrating tablet (ODT) composition of claim 14 comprising microparticles of a drug and/or a pharmaceutically acceptable salt, solvate coated with one or more taste-masking membranes, exhibit one or more of the following properties:
i. acceptable hardness and friability suitable for packaging in bottles and blister packaging, storage, transportation, commercial distribution, and end use;
ii. disintegration on contact with saliva in the oral cavity in 60 seconds forming a smooth, easy-to-swallow suspension with a pleasant taste (no grittiness or aftertaste), meeting the specification of not more than 30 seconds in the <USP 701> Disintegration Test;
iii. taste-masked drug particles exhibit smooth mouthfeel (non- gritty) and no aftertaste;
iv. provide rapid, substantially-complete release of the dose upon entry into the stomach, as evident by meeting the dissolution specifications of about 85% of the dose in about 45 minutes in 900 mL of 0.01N HC1 buffer when tested for dissolution using USP Apparatus 2 (paddles @ 75 rpm);
v. enhances the probability of achieving bioequivalence to reference listed drug, non-ODT product of equal strength, patient convenience and compliance with the dosing regimen.

16. A method of manufacturing a taste-masked composition comprising the steps of:
a.
(i) applying one or more polymeric membranes onto drug particles containing a drug and/or a pharmaceutically acceptable salt, solvate, polymorph, or ester thereof, with a desired particle size distribution; or
(ii) applying a first coating layer comprising a water-insoluble polymeric material for a weight gain of from 5% w/w to 20% w/w, onto microparticles containing a drug and/or a pharmaceutically acceptable salt, solvate, polymorph, or ester thereof, with desired particle size specifications; and
applying an optional second coating layer comprising a water insoluble enteric polymer blend material at a ratio of water insoluble polymer to enteric polymer of from 9: 1 to 1 :2 for a weight gain of from 10% w/w to 40% w/w, onto drug particles
b. a compressible coating with a non-polymeric material directly disposed over the modified-release coated drug particles of step (a);
c. producing rapidly dispersing granules with an averge particle size of not more than 400 µm, comprising a sugar alcohol, a saccharide, or a mixture thereof, and a super disintegrant with an average particle size of not more than 30 pm, by granulating said mixture at a ratio of from 99/1 to 90/10 using water as a granulating fluid and drying in a fluid bed dryer or in a tray drying;
d. blending compressible coated taste-masked drug particles from (b), rapidly dispersing microgranules from (c) and additional excipients comprising one or more flavoring agents, one or more colorants, a sweetener, additional disintegrant, and a diluent such as microcrystalline cellulose; and
e. compressing the blend from (d) into orally disintegrating tablets using a rotary tablet press equipped with an external lubrication system,
wherein
(1) one or more said taste-masking layers effectively mask the taste of said drug and/or the pharmaceutically acceptable salt, ester, or solvate thereof,
(2) said orally disintegrating tablet rapidly disintegrates in the oral cavity of a patient into a smooth, easy-to-swallow suspension containing taste-masked particles exhibiting non-gritty mouthfeel and no aftertaste.

17. The method for manufacturing a modified-release composition, in accordance with claim 16 wherein manufacturing microparticles comprising taste-masking or modified-release coatings disposed over drug particles further comprises the steps of applying a compressible coating layer comprising a non-polymeric sweetener selected from the group consisting of sucralose, lactitol, sorbitol, maltitol, or a mixture thereof, onto taste-masked or modified-release coated drug particles.

## Patentansprüche

1. Eine pharmazeutische Zusammensetzung, die komprimierbare beschichtete Mikropartikel beinhaltet, beinhaltend:
a. Wirkstoffpartikel, die einen Wirkstoff und/oder ein pharmazeutisch akzeptables Salz, einen Ester, ein Polymorph oder Solvat davon beinhalten;
b. mindestens eine Schicht einer Membran mit modifizierter Freisetzung, die ein wasserunlösliches polymeres Material beinhaltet, das über den wirkstoffhaltigen Kernen angeordnet ist; und
c. eine komprimierbare Überzugsschicht, die einen nicht polymeren Süßstoff beinhaltet, der direkt über den beschichteten Wirkstoffpartikeln mit modifizierter Freisetzung von (b) angeordnet ist.

2. Pharmazeutische Zusammensetzung gemäß Anspruch 1, wobei die Membranschicht mit modifizierter Freisetzung zum Erzielen eines Gewichtszuwachses von 5 bis 50 Gewichts-%, bezogen auf das Gesamtgewicht der komprimierbaren beschichteten Mikropartikel, appliziert wird.

3. Pharmazeutische Zusammensetzung gemäß Anspruch 1, wobei die Membran mit modifizierter Freisetzung eine geschmacksmaskierende Schicht ist, wobei die geschmacksmaskierende Schicht zum Erzielen eines Gewichtszuwachses von 5 bis 50 Gewichts-%, bezogen auf das Gesamtgewicht der komprimierbaren beschichteten Mikropartikel, appliziert wird.

4. Pharmazeutische Zusammensetzung gemäß Anspruch 3, wobei die geschmacksmaskierende Schicht ferner einen magenlöslichen Porenbildner beinhaltet, wobei das Verhältnis des wasserunlöslichen polymeren Materials zu dem magenlöslichen Porenbildner in der Schicht von 95 : 5 bis 50 : 50 variiert und die gesamte Schicht zum Erzielen eines Gewichtszuwachses von 5 bis 50 Gewichts-%, bezogen auf das Gesamtgewicht der komprimierbaren beschichteten Mikropartikel, appliziert wird, und wobei die geschmacksmaskierende Schicht den Geschmack des Wirkstoffs und/oder pharmazeutisch akzeptablen Salzes, Esters oder Solvats davon wirksam maskiert und eine im Wesentlichen vollständige Freisetzung der Dosis nach Eintritt in den Magen bereitstellt.

5. Pharmazeutische Zusammensetzung gemäß Anspruch 3, die ferner eine Abdichtungsschicht beinhaltet, die ein hydrophiles Polymer beinhaltet, das über dem wirkstoffhaltigen Kernpartikel angeordnet ist, wobei die Abdichtungsschicht zum Erzielen eines Gewichtszuwachses von 1 bis 10 Gewichts-%, bezogen auf das Gesamtgewicht des komprimierbaren beschichteten Mikropartikels, appliziert wird.

6. Pharmazeutische Zusammensetzung gemäß Anspruch 3, die ferner eine zweite Membranschicht beinhaltet, die ein wasserunlösliches Polymer in Kombination mit einem magenlöslichen porenbildenden Polymer beinhaltet, die über der geschmacksmaskierenden Schicht angeordnet ist, wobei die zweite Membranschicht zum Erzielen eines Gewichtszuwachses von 5 bis 50 Gewichts-%, bezogen auf das Gesamtgewicht des komprimierbaren beschichteten Mikropartikels, appliziert wird und wobei der erste und/oder zweite Überzug im Wesentlichen den Geschmack des wirkstoffhaltigen Kernpartikels maskiert.

7. Pharmazeutische Zusammensetzung gemäß Anspruch 3, wobei die pharmazeutische Zusammensetzung mindestens 70% des Wirkstoffs oder eines pharmazeutisch akzeptablen Salzes, Esters, Solvats oder einer Kombination davon freisetzt, nachdem sie in den Magen eintritt oder innerhalb von 30 Minuten, wenn sie auf Lösung in simuliertem Magensaft oder 0,01 N HCl in dem United-States-Pharmacopoeia-Apparat 2 getestet wird (Paddel bei 50 U/min in 900 ml Puffer mit einem pH-Wert von 1,2 oder 2,0).

8. Pharmazeutische Zusammensetzung gemäß Anspruch 1, die ferner eine Abdichtungsschicht beinhaltet, die ein hydrophiles Polymer beinhaltet, das über dem wirkstoffhaltigen Kernpartikel angeordnet ist.

9. Pharmazeutische Zusammensetzung gemäß Anspruch 1, wobei die beschichtete Membranschicht mit modifizierter Freisetzung zum Erzielen eines Gewichtszuwachses von 5 bis 25 Gewichts-%, bezogen auf das Gesamtgewicht des komprimierbaren beschichteten Mikropartikels, appliziert wird.

10. Pharmazeutische Zusammensetzung gemäß Anspruch 9, wobei die pharmazeutische Zusammensetzung ein hinhaltendes Freisetzungsprofil des Wirkstoffs oder eines pharmazeutisch akzeptablen Salzes, Esters, Solvats oder einer Kombination davon bereitstellt, nach Eintritt in den Magen-Darm-Trakt oder wenn sie auf Lösung in dem United-States-Pharmacopoeia-Apparat 2 getestet wird (Paddel bei 50 U/min bei 37°C in 700 ml Puffer mit einem pH-Wert von 1,2 für die ersten 2 Stunden, gefolgt von weiteren Tests bei einem pH-Wert von 6,8, erhalten durch Zugabe von 200 ml eines pH-Modifikators).

11. Pharmazeutische Zusammensetzung gemäß Anspruch 9, wobei die Membranschicht ferner ein magensaftresistentes Polymer beinhaltet, wobei das Verhältnis des wasserunlöslichen polymeren Materials zu dem magensaftresistenten Polymer in der ersten Membran von 9 : 1 bis 1 : 2 variiert und die gesamte Membran zum Erzielen eines Gewichtszuwachses von 10 bis 40 Gewichts-%, bezogen auf das Gesamtgewicht des komprimierbaren beschichteten Mikropartikels, appliziert wird.

12. Pharmazeutische Zusammensetzung gemäß Anspruch 9, die ferner eine zweite Membran beinhaltet, wobei die zweite Membran ein wasserunlösliches Polymer in Kombination mit einem magensaftresistenten Polymer beinhaltet, wobei das Verhältnis des wasserunlöslichen Polymers zu dem magensaftresistenten Polymer in der zweiten Membran von 9 : 1 bis 1 : 2 variiert und die gesamte Membran zum Erzielen eines Gewichtszuwachses von 10 bis 40 Gewichts-%, bezogen auf das Gesamtgewicht des komprimierbaren beschichteten Mikropartikels, appliziert wird.

13. Pharmazeutische Zusammensetzung gemäß Anspruch 1, wobei die beschichteten Wirkstoffmikropartikel mit modifizierter Freisetzung eine mittlere Partikelgröße von 500 µm oder weniger aufweisen.

14. Eine Schmelztablettenzusammensetzung (Schmelztablette, ODT) oder eine rasch zerfallende Tablettenzusammensetzung (rapidly dispersing, RDT), die geschmacksmaskierte und/oder beschichtete wirkstoffhaltige Kerne mit modifizierter Freisetzung gemäß Anspruch 1 oder 9 beinhaltet, beinhaltend:
a. Wirkstoffpartikel, die eine therapeutisch wirksame Menge eines Wirkstoffs und/oder eines pharmazeutisch akzeptablen Salzes, Esters oder Solvats davon beinhalten;
b. eine oder mehrere polymere Membranen, wobei jede Membran ein wasserunlösliches Polymer oder optional ein wasserunlösliches und magenlösliches polymeres Mischmaterial beinhaltet;
c. einen komprimierbaren Überzug mit einem nicht polymeren Süßstoff, ausgewählt aus der Gruppe, bestehend aus Sucralose, Lactitol, Sorbitol, Maltitol oder einem Gemisch davon, der direkt über den beschichteten Wirkstoffpartikeln mit modifizierter Freisetzung von Schritt (b.) angeordnet ist; und
d. rasch zerfallende Granulae, beinhaltend (i) ein Sprengmittel und (ii) einen Zuckeralkohol, ein Saccharid oder ein Gemisch davon, in einem Verhältnis von 90 : 10 bis 99 : 1.

15. Schmelztablettenzusammensetzung (ODT) gemäß Anspruch 14, die Mikropartikel eines Wirkstoffs und/oder eines pharmazeutisch akzeptablen Salzes, Solvats beinhalten, beschichtet mit einer oder mehreren geschmacksmaskierenden Membranen, die eine oder mehrere der folgenden Eigenschaften aufweisen:
i. akzeptable Härte und Bröckeligkeit, die für das Verpacken in Flaschen und Blisterpackungen, die Lagerung, den Transport, den kommerziellen Vertrieb und Endverbrauch geeignet sind;
ii. Zerfall bei Kontakt mit Speichel in der Mundhöhle in 60 Sekunden mit Bilden einer glatten, leicht zu schluckenden Suspension mit einem angenehmen Geschmack (ohne Sandigkeit oder Nachgeschmack), die die Spezifikation von nicht mehr als 30 Sekunden in dem <USP-701>-Zerfallstest erfüllt;
iii. geschmacksmaskierte Wirkstoffpartikel zeigen ein glattes Gefühl im Mund (nicht sandig) und haben keinen Nachgeschmack;
iv. stellen eine rasche, im Wesentlichen vollständige Freisetzung der Dosis nach Eintritt in den Magen bereit, was durch das Erfüllen der Lösungsspezifikationen von etwa 85% der Dosis in etwa 45 Minuten in 900 ml 0,01-N-HC1-Puffer beim Testen auf Lösung unter Verwendung des USP-Apparat 2 (Paddel bei 75 U/min) ersichtlich ist;
v. erhöht die Wahrscheinlichkeit, dass Bioäquivalenz mit dem als Referenz aufgeführten Wirkstoff-nicht-ODT-Produkt, das die gleiche Stärke aufweist, Bequemlichkeit für den Patienten und Compliance mit dem Dosierungsschema erzielt werden.

16. Ein Verfahren zur Herstellung einer geschmacksmaskierten Zusammensetzung, das die folgenden Schritte beinhaltet:
a.
(i) Applizieren von einer oder mehreren polymeren Membranen auf Wirkstoffpartikel, die einen Wirkstoff und/oder ein pharmazeutisch akzeptables Salz, Solvat, Polymorph oder einen Ester davon enthalten, mit einer erwünschten Partikelgrößenverteilung; oder
(ii) Applizieren einer ersten Überzugsschicht, die ein wasserunlösliches polymeres Material beinhaltet, zum Erzielen eines Gewichtszuwachses von 5 Gew.-% bis 20 Gew.-% auf Mikropartikel, die einen Wirkstoff und/oder ein pharmazeutisch akzeptables Salz, Solvat, Polymorph oder einen Ester davon enthalten, mit erwünschten Partikelgrößenspezifikationen; und
Applizieren einer optionalen zweiten Überzugsschicht, die ein wasserunlösliches magensaftresistentes Polymermischmaterial in einem Verhältnis des wasserunlöslichen Polymers zu dem magensaftresistenten Polymer von 9 : 1 bis 1 : 2 beinhaltet, zum Erzielen eines Gewichtszuwachses von 10 Gew.-% bis 40 Gew.-% auf Wirkstoffpartikel
b. einen komprimierbaren Überzug mit einem nicht polymeren Material, der direkt über den beschichteten Wirkstoffpartikeln mit modifizierter Freisetzung von Schritt (a) angeordnet ist;
c. Herstellen rasch zerfallender Granulae mit einer mittleren Partikelgröße von nicht mehr als 400 pm, beinhaltend einen Zuckeralkohol, ein Saccharid oder ein Gemisch davon, und ein Supersprengmittel mit einer mittleren Partikelgröße von nicht mehr als 30 pm, durch Granulieren des Gemischs bei einem Verhältnis von 99 : 1 bis 90 : 10 unter Verwendung von Wasser als Granulierfluid und Trocknen in einem Wirbelschichttrockner oder Hordentrocknen;
d. Mischen der komprimierbaren beschichteten geschmacksmaskierten Wirkstoffpartikel von (b), der rasch zerfallenden Mikrogranulae von (c) und zusätzlicher Hilfsstoffe, die einen oder mehrere Aromastoffe, einen oder mehrere Farbstoffe, einen Süßstoff, zusätzliches Sprengmittel und ein Verdünnungsmittel wie etwa mikrokristalline Cellulose beinhalten; und
e. Komprimieren des Gemischs von (d) zu Schmelztabletten unter Verwendung einer Rundläufer-Tablettenpresse, die mit einem externen Schmiersystem ausgestattet ist,
wobei
(1)die eine oder mehreren geschmacksmaskierenden Schichten den Geschmack des Wirkstoffs und/oder des pharmazeutisch akzeptablen Salzes, Esters oder Solvats davon wirksam maskieren,
(2)die Schmelztablette rasch in der Mundhöhle eines Patienten zu einer glatten, leicht zu schluckenden Suspension zerfällt, die geschmacksmaskierte Partikel enthält, die ein nicht sandiges Gefühl im Mund zeigen und keinen Nachgeschmack haben.

17. Verfahren zur Herstellung einer Zusammensetzung mit modifizierter Freisetzung gemäß Anspruch 16, wobei das Fertigen von Mikropartikeln, die geschmacksmaskierende oder Überzüge mit modifizierter Freisetzung beinhalten, die über Wirkstoffpartikeln angeordnet sind, ferner die folgenden Schritte beinhaltet: Applizieren einer komprimierbaren Überzugsschicht, beinhaltend einen nicht polymeren Süßstoff, ausgewählt aus der Gruppe, bestehend aus Sucralose, Lactitol, Sorbitol, Maltitol oder einem Gemisch davon, auf geschmacksmaskierte oder beschichtete Wirkstoffpartikel mit modifizierter Freisetzung.

## Revendications

1. Composition pharmaceutique comprenant des microparticules enrobées et compressibles comprenant :
a. des particules de médicament comprenant un médicament et/ou son sel, ester, polymorphe ou solvate pharmaceutiquement acceptable ;
b. au moins une couche de membrane à libération modifiée comprenant une matière polymérique insoluble dans l'eau disposée sur les noyaux contenant un médicament ; et
c. une couche d'enrobage compressible comprenant un édulcorant non polymérique, disposé directement sur les particules de médicament enrobées à libération modifiée de (b).

2. Composition pharmaceutique selon la revendication 1, dans laquelle ladite couche de membrane à libération modifiée est appliquée pour un gain pondéral de 5 % à 50 % en poids par rapport au poids total des microparticules enrobées et compressibles.

3. Composition pharmaceutique selon la revendication 1, dans laquelle ladite membrane à libération modifiée est une couche de masquage de goût, dans laquelle ladite couche de masquage de goût est appliquée pour un gain pondéral de 5 % à 50 % en poids par rapport au poids total des microparticules enrobées et compressibles.

4. Composition pharmaceutique selon la revendication 3, dans laquelle ladite couche de masquage de goût comprend en outre un agent porogène gastrosoluble, dans laquelle le rapport entre la matière polymérique insoluble dans l'eau et l'agent porogène gastrosoluble dans ladite couche varie de 95:5 à 50:50 et la couche entière est appliquée pour un gain pondéral de 5 % à 50 % en poids par rapport au poids total des microparticules enrobées et compressibles, et dans laquelle ladite couche de masquage de goût masque efficacement le goût du médicament et/ou de son sel, ester, ou solvate pharmaceutiquement acceptable et permet la libération pratiquement complète de la dose lors de l'introduction dans l'estomac.

5. Composition pharmaceutique selon la revendication 3, comprenant en outre une couche d'étanchéité comprenant un polymère hydrophile disposé sur ladite particule centrale contenant un médicament, dans laquelle ladite couche d'étanchéité est appliquée pour un gain pondéral de 1 % à 10 % en poids par rapport au poids total des microparticules enrobées et compressibles.

6. Composition pharmaceutique selon la revendication 3, comprenant en outre une seconde couche de membrane comprenant un polymère insoluble dans l'eau associé à un polymère porogène gastrosoluble disposé sur ladite couche de masquage de goût, dans laquelle ladite seconde couche de membrane est appliquée pour un gain pondéral de 5 % à 50 % en poids par rapport au poids total des microparticules enrobées et compressibles, et dans laquelle le(s)dit(s) premier et/ou second enrobage(s) masque(nt) pratiquement le goût de la particule centrale contenant un médicament.

7. Composition pharmaceutique selon la revendication 3, dans laquelle ladite composition pharmaceutique libère au moins 70 % dudit médicament ou de son sel, ester, solvate pharmaceutiquement acceptable, ou d'une combinaison de ceux-ci, lors de l'introduction dans l'estomac ou dans les 30 minutes lors d'un test de dissolution dans un liquide gastrique simulé ou HCl 0,01 N selon la Pharmacopée des États-Unis - Apparatus 2 (palettes à 50 tr/min dans 900 mL de tampon à pH 1,2 ou pH 2,0).

8. Composition pharmaceutique selon la revendication 1, comprenant en outre une couche d'étanchéité comprenant un polymère hydrophile disposé sur ladite particule centrale contenant un médicament.

9. Composition pharmaceutique selon la revendication 1, dans laquelle ladite couche de membrane enrobée à libération modifiée est appliquée pour un gain pondéral de 5 % à 25 % en poids par rapport au poids total des microparticules enrobées et compressibles.

10. Composition pharmaceutique selon la revendication 9, dans laquelle ladite composition pharmaceutique fournit un profil à libération prolongée dudit médicament ou d'un sel, ester, solvate pharmaceutiquement acceptable, ou d'une combinaison de ceux-ci, lors de l'introduction dans le tractus gastro-intestinal ou lors d'un test de dissolution selon la Pharmacopée des États-Unis - Apparatus 2 (palettes à 50 tr/min à 37 °C dans 700 mL de tampon à pH 1,2 pendant les 2 premières heures, puis test supplémentaire à pH 6,8 obtenu par l'ajout de 200 mL d'un modificateur de pH).

11. Composition pharmaceutique selon la revendication 9, dans laquelle ladite couche de membrane comprend en outre un polymère entérique, dans laquelle le rapport entre la matière polymérique insoluble dans l'eau et le polymère entérique dans ladite première membrane varie de 9:1 à 1:2 et la membrane entière est appliquée pour un gain pondéral de 10 % à 40 % en poids par rapport au poids total des microparticules enrobées et compressibles.

12. Composition pharmaceutique selon la revendication 9, comprenant en outre une seconde membrane, dans laquelle la seconde membrane comprend un polymère insoluble dans l'eau associé à un polymère entérique, dans laquelle le rapport entre le polymère insoluble dans l'eau et le polymère entérique dans ladite seconde membrane varie de 9:1 à 1:2 et la membrane entière est appliquée pour un gain pondéral de 10 % à 40 % en poids par rapport au poids total des microparticules enrobées et compressibles.

13. Composition pharmaceutique selon la revendication 1, dans laquelle lesdites microparticules de médicament enrobées à libération modifiée ont une dimension moyenne de particule de 500 µm ou moins.

14. Composition de comprimé à délitement buccal (ODT) ou de comprimé à dispersion rapide (RDT) comprenant des noyaux enrobés contenant un médicament à goût masqué et/ou à libération modifiée selon la revendication 1 ou 9 comprenant :
a. des particules de médicament comprenant une quantité thérapeutiquement efficace d'un médicament et/ou de son sel, ester, ou solvate pharmaceutiquement acceptable ;
b. une ou plusieurs membranes polymériques, chaque membrane comprenant un polymère insoluble dans l'eau ou éventuellement une matière de mélange polymérique insoluble dans l'eau et gastrosoluble ;
c. un enrobage compressible avec un édulcorant non polymérique choisi dans le groupe constitué par le sucralose, le lactitol, le sorbitol, le maltitol, ou leur mélange, directement disposé sur les particules de médicament enrobées à libération modifiée de l'étape (b.) ; et
d. des granulés à dispersion rapide comprenant (i) un agent délitant et (ii) un alcool de sucre, un saccharide, ou leur mélange, dans un rapport allant de 90:10 à 99:1.

15. Composition de comprimé à délitement buccal (ODT) selon la revendication 14, comprenant des microparticules d'un médicament et/ou d'un sel, d'un solvate pharmaceutiquement acceptables, enrobées avec une ou plusieurs membranes de masquage de goût, présentant une ou plusieurs des propriétés suivantes :
i. dureté et friabilité acceptables adaptées au conditionnement en flacons et emballage plaquette, au stockage, au transport, à la distribution commerciale, et à l'utilisation finale ;
ii. désintégration au contact de la salive dans la cavité buccale en 60 secondes, formant une suspension fluide, facile à avaler, au goût agréable (aucune contexture sableuse ou aucun arrière-goût), conforme aux spécifications de 30 secondes au plus dans le test de désintégration <USP 701> ;
iii. particules de médicament à goût masqué présentant une sensation fluide en bouche (non sableuse) et aucun arrière-goût ;
iv. assurance d'une libération rapide, pratiquement complète de la dose lors de l'introduction dans l'estomac, comme en témoigne la conformité avec les spécifications de dissolution d'environ 85 % de la dose en environ 45 minutes dans 900 mL de tampon HCl 0,01 N lors d'un test de dissolution selon USP - Apparatus 2 (palettes à 75 tr/min) ;
v. augmentation de la probabilité de parvenir à une bioéquivalence avec un médicament de la liste de référence, un produit non ODT de même concentration, au confort du patient et à l'observance du schéma posologique.

16. Procédé de fabrication d'une composition à goût masqué comprenant les étapes comprenant :
a.
(i) application d'une ou de plusieurs membranes polymériques sur les particules de médicament contenant un médicament et/ou son sel, solvate, polymorphe, ou ester pharmaceutiquement acceptable, avec une distribution granulométrique souhaitée ; ou
(ii) application d'une première couche d'enrobage comprenant une matière polymérique insoluble dans l'eau pour un gain pondéral de 5 % poids/poids à 20 % poids/poids, sur les microparticules contenant un médicament et/ou son sel, solvate, polymorphe, ou ester pharmaceutiquement acceptable, avec les spécifications de dimension de particule souhaitées ; et
application d'une seconde couche d'enrobage facultative comprenant une matière de mélange polymérique entérique insoluble dans l'eau dans un rapport entre le polymère insoluble dans l'eau et le polymère entérique allant de 9:1 à 1:2 pour un gain pondéral de 10 % poids/poids à 40 % poids/poids, sur les particules de médicament
b. un enrobage compressible avec une matière non polymérique directement disposée sur les particules de médicament enrobées à libération modifiée de l'étape (a) ;
c. production de granulés à dispersion rapide avec une dimension moyenne de particule égale ou inférieure à 400 pm, comprenant un alcool de sucre, un saccharide, ou leur mélange, et un superdélitant avec une dimension moyenne de particule égale ou inférieure à 30 pm, par granulation dudit mélange dans un rapport allant de 99/1 à 90/10, en utilisant de l'eau comme liquide de granulation, et séchage dans un sécheur à lit fluidisé ou dans un processus de dessiccation sur plateaux ;
d. mélange des particules de médicament enrobées et compressibles à goût masqué de (b), des microgranulés à dispersion rapide de (c) et d'excipients supplémentaires comprenant un ou plusieurs aromatisants, un ou plusieurs colorants, un édulcorant, un délitant supplémentaire, et un diluant tel que la cellulose microcristalline ; et
e. compression du mélange de (d) en comprimés à délitement buccal au moyen d'une presse rotative à comprimés équipée d'un système de lubrification externe,
dans lequel
(1) une ou plusieurs desdites couches de masquage de goût masquent efficacement le goût dudit médicament et/ou de son sel, ester, ou solvate pharmaceutiquement acceptable,
(2) ledit comprimé à délitement buccal se désintègre rapidement dans la cavité buccale d'un patient en une suspension fluide, facile à avaler, contenant des particules à goût masqué présentant une sensation en bouche non sableuse et n'ayant aucun arrière-goût.

17. Procédé de fabrication d'une composition à libération modifiée selon la revendication 16, dans lequel la fabrication de microparticules comprenant des enrobages à masquage de goût ou à libération modifiée disposés sur des particules de médicament comprend en outre les étapes d'application d'une couche d'enrobage compressible comprenant un édulcorant non polymérique choisi dans le groupe constitué par le sucralose, le lactitol, le sorbitol, le maltitol, ou leur mélange, sur des particules de médicament enrobées à goût masqué ou à libération modifiée.
